# EUROPEAN PATENT SPECIFICATION

(11) **EP 3 476 385 B1**
(45) Date of publication and mention of the grant of the patent: **29.11.2023**
(21) Application number: 17198740.7
(22) Date of filing: 27.10.2017
(51) Int. Cl.: A61K 9/00, A61K 9/70

(54) **COLLAPSIBLE MYOCARDIAL PATCH**
ZUSAMMENKLAPPBARER MYOKARD-PATCH
TIMBRE MYOCARDIQUE PLIABLE

(43) Date of publication of application: 01.05.2019
(73) Proprietor: AdjuCor GmbH, 81673 München (DE)
(72) Inventor: WILDHIRT, Stephen, 80997 Munich (DE); MAIER, Andreas, 85567 Grafing (DE); HOFMANN, Björn, 85661 Forstinning (DE); DE VAAL, Hamman, 80689 Munich (DE)
(74) Representative: Peterreins Schley

(56) References cited:
- US-A1- 2003 195 527
- US-B1- 7 922 648

## Description

### Technical Field

The present invention relates to a collapsible myocardial patch for site-specific delivery of a therapeutic agent to an epicardial surface of a heart. Furthermore, the invention relates to a kit comprising such a collapsible myocardial patch and a medical device for introduction of the patch into the pericardial sac or a therapeutic agent for use in treating a heart condition.

### Background

The delivery of therapeutic agent to a specific treatment site represents a substantial challenge in the design of drug delivery systems. While therapeutic agents designed for action at or within the heart may be suitable for systemic delivery, the amount of therapeutic agent that must be delivered by this route generally must be quite high in order to result in delivery of a therapeutically effective amount at the desired site of action. Delivery of such high amounts of therapeutic agent increases the likelihood and severity of side effects. In addition, some therapeutic agents cannot be delivered systemically. Therefore, localized delivery of a therapeutic agent is often preferable or even necessary. In many instances, it is further preferable or necessary to locally deliver the therapeutic agent to the outside of the heart, i.e. to the epicardial surface of the heart.

Delivery of therapeutic agents to the epicardial surface of the heart typically involves delivery via the pericardial space. The pericardium, or pericardial sac, is a membranous sac in which the heart is contained. The pericardium and the pericardial fluid together function to prevent excessive dilation of the chambers of the heart during normal activity, to lubricate the surfaces of the heart, and to maintain the heart in a fixed geometric position. The space between the pericardium and the heart, known as the pericardial space, contains the pericardial fluid which bathes the heart. However, while it may be viable for some therapeutic agents to treat the epicardial surface of a heart by administering the therapeutic agent into the pericardial space, it is often desirable to additionally ensure localized delivery to the epicardial surface while limiting the exposure of the pericardial sac and other epicardial regions to the therapeutic agent.

US 6,726,920 B1 discloses a delivery system for targeted delivery of a drug to the epicardial surface while minimizing the exposure of the pericardial sac to the drug. The delivery system includes a myocardial patch that is adhered to the epicardial surface of the heart. The patch contains a reservoir lumen that selectively releases a drug to the epicardial surface. This is achieved by providing the patch with a backing layer in the direction of the pericardial sac that is impermeable to the drug. US 6,726,920 B1 further discloses to adhere the patch to the heart by providing the patch with an adhesive layer on the patch surface facing towards the heart. In practice, such a patch has limited utility since the amount of adhesive required to securely affix the patch to the heart is rather high. This implies that the adhesive layer is either very thick resulting a very bulky patch and, in consequence, difficult to deliver to the heart, or a compromise between drug safety and efficacy (by risking migration of the patch after deployment due to insufficient attachment to the heart) and ease of deliverability.

Even without delivery of a therapeutic agent to the epicardial surface, just the presence of an epicardial patch made of a biodegradable, elastomeric material has been demonstrated to additionally provide a substantial amount of mechanical support to ameliorate the dilated effect that would normally ensue following myocardial infarction, as disclosed in US 8,974,542 B2. However, this model of implanting an epicardial patch relies on the suturing of the patch continuously around its edges to the heart surface, which makes it unsuitable for minimally invasive procedures and adds significant risk due to the need for suturing and increased invasiveness.

In conclusion, it would be desirable to have a drug delivery system that is easy to deliver to the heart and localizes a therapeutic agent at a specific therapy site.

US 2003/195527 A1 discloses to system and method for the delivery of therapeutic agents, such as therapeutic drugs or genetic material, into the muscle or tissue that minimizes the loss of therapeutic agents due to contraction of the muscle.

US 7,922,648 B1 discloses a myocardial infarction patch for a a minimally invasive implant.

### Summary

The present invention relates to a collapsible myocardial patch for site-specific delivery of a therapeutic agent to an epicardial surface of a heart. Furthermore, the disclosure describes a kit comprising such a collapsible myocardial patch and a medical device for introduction of the patch into the pericardial sac or a therapeutic agent for use in treating a heart condition.

More specifically, the present invention relates to a patch that may be less bulky and, thus, easier to deliver to the heart. At the same time, the patch may be less prone to migration after deployment. These benefits may be realized by providing a myocardial patch that lacks an adhesive during the initial phase of patch deployment. Therefore, the patch may be less bulky, easier to deploy and maneuver at the heart, and deliverable per smaller and, thus, less invasive delivery systems, such as catheters. It was found that the patch of the present invention partially self-adheres to the heart to add stability in aid of the subsequent deployment of an adhesive to securely and permanently adhere the patch to the heart. The initial self-adherence of the patch may be further aided by the pericardial sac pressing the patch against the surface of the heart. Supplying the adhesive to the patch only after delivering the patch also allows using more reactive two component adhesives which provide a stronger bond to the heart and allow transport of the adhesive over a certain conduit length in its temporary pre-polymerized state, thereby enabling extracorporeal administration of the adhesive. Such fast acting two component adhesives would already be polymerized if they were extracorporeally applied to a patch prior to its deployment and, therefore, are not usable on the patches disclosed in US 6,726,920 B1 without drastically increasing gel times beyond values acceptable for securely adhering the patch to a living heart and reducing bonding strength.

In a first aspect, the present invention relates to a collapsible myocardial patch for site-specific delivery of a therapeutic agent to an epicardial surface of a heart having a collapsed and an expanded state, as disclosed in the appended claims. The collapsible myocardial patch comprises a first layer and a second layer. The first layer and the second layer define a delivery side and a cover side of the myocardial patch in its expanded state, respectively. Furthermore, the collapsible myocardial patch comprises at least one reservoir lumen, which is defined by the first and/or second layer and whereby the reservoir lumen is configured to contain a therapeutic agent. The collapsible myocardial path also comprises at least one bordering lumen which is defined by the first and/or second layer. Thereby, the bordering lumen is different from the reservoir lumen and configured to contain and release an adhesive material. Additionally, the collapsible myocardial path comprises at least one port which is in fluid communication with at least one of the bordering lumen and configured to receive the adhesive material. Therein, the first and the second layer are configured to preferentially release the therapeutic agent towards the delivery side of the patch in its expanded state.

In another aspect, which is combinable with the previous aspect, the at least one bordering lumen may be in a peripheral region on the patch. Alternatively, or additionally, the at least one bordering lumen may be configured to release the adhesive material towards the delivery side of the patch in its expanded state. Additionally, the at least one bordering lumen may comprise one or more openings arranged o the delivery side of the patch in its expanded state. Alternatively, or additionally, the first layer may be permeable to the therapeutic agent and the second layer may be less permeable to the therapeutic agent than the first layer.

In another aspect, which is combinable with any one of the previous aspects, the first layer may be biodegradable under in-situ conditions and the second layer may be more slowly biodegrading than the first layer under said in-situ conditions.

In another aspect, which is combinable with any one of the previous aspects, the first and the second layer may join adjacent to one or more bordering regions and may define the reservoir lumen. Alternatively, the first and the second layer may be connected to one or more bordering regions. Thereby, the first and the second layer and the one or more bordering regions may define the reservoir lumen.

In another aspect, which is combinable with any one of the previous aspects, the reservoir lumen may contain the therapeutic agent and a carrier material. Alternatively, the reservoir lumen may be in fluid communication with a second port which may be configured to receive the therapeutic agent and a carrier material.

In another aspect, wherein the at least one bordering lumen is configured to release the adhesive material towards the epicardial surface of the heart in the expanded state of the patch and wherein the at least one bordering lumen comprises one or more openings facing towards the epicardial surface in the expanded state of the patch, the one or more openings in the bordering lumen may be a plurality of holes, slits or pores which may be configured to allow passage of the adhesive material. Additionally, the cross-sectional area of each of said plurality of holes, slits or pores may be larger or identical to the cross-sectional area of a more proximal one of said plurality of holes, slits or pores.

In another aspect, which is combinable with any one of the previous aspects, the one or more bordering lumen may occupy at least 10% of the circumference of the patch.

In another aspect, which is combinable with any one of the previous aspects, at least one of the bordering lumen may define one or more channels. Additionally, at least one of the channels may be located at or running along the circumference of the patch.

In another aspect, which is combinable with any one of the previous aspects, the patch substantially may have a shape selected from the group consisting of: a circular shape, an oval shape, a rectangular shape, a triangular shape, a trapezoidal shape, a double-triangular shape, a double-trapezoidal shape, and a star-like shape.

In another aspect, which is combinable with any one of the previous aspects, the patch may be three-dimensionally adapted to substantially conform to the shape of the heart in the expanded state of the patch. Additionally, the patch may have a substantially circular or oval convex shape. "Substantially" in this context may refer to a slight geometric deviation from the mentioned shape which does not negate the function of the device and/or feature in question.

In another aspect, which is combinable with any one of the previous aspects, at least part of its surface may comprise a feature that facilitates tissue ingrowth and/or fibrosis. Additionally, the feature facilitating tissue ingrowth and/or fibrosis is arranged on the delivery side of the patch in its expanded state.

In another aspect, which is combinable with the previous aspect, said feature may be one or more features selected from the group consisting of:
a) a layer of non-woven fibrous material that covers at least in part the surface area of the reservoir lumen,
b) a layer of non-woven fibrous material that covers at least in part the surface area of the patch adjacent to the reservoir lumen,
c) an irregular surface morphology that facilitates tissue ingrowth and/or fibrosis that covers at least in part the surface area of the reservoir lumen,
d) an irregular surface morphology that facilitates tissue ingrowth and/or fibrosis that covers at least in part the surface area of the patch adjacent to the reservoir lumen,
e) a layer of open- or closed-cell foam material that covers at least in part the surface area of the reservoir lumen,
f) a layer of open- or closed-cell foam material that covers at least in part the surface area of the patch adjacent to the reservoir lumen,
g) a second therapeutic agent that facilitates tissue ingrowth and/or fibrosis that covers at least in part the surface area of the reservoir lumen, and
h) a second therapeutic agent that facilitates tissue ingrowth and/or fibrosis that covers at least in part the surface area of the patch adjacent to the reservoir lumen.

In another aspect, which is combinable with any one of the previous aspects, the patch may be releasably attached to a support structure which provides one or more of the following features:
a) an inflatable member which is configured to expand the myocardial patch from its collapsed state into an expanded state,
b) a shape memory material which is configured to expand the myocardial patch from its collapsed state into an expanded state,
c) a hollow member which is configured to receive a catheter wire or stylet which expands the myocardial patch from its collapsed state into an expanded state, and
d) a flexible foam material which is configured to expand the myocardial patch from its collapsed state into an expanded state.

Additionally, the patch may be releasably attached to the support structure on the cover side of the patch in its expanded state.

In another aspect, which is combinable with any one of the previous aspects, the patch may be configured to allow its passage through a catheter or port having an internal diameter of less than about 15 mm, in particular less than about 11 mm, in its collapsed state.

In another aspect, which is combinable with any one of the previous aspects, the patch may be biodegradable within a time period of less than 2 years, preferably between 2 weeks and 6 months.

In another aspect, which is combinable with any one of the previous aspects, the patch may comprise a biodegradable foam, in particular a biodegradable polyester urethane foam or a biodegradable polyester urea urethane foam.

In another aspect, which is combinable with any one of the previous aspects, the patch may weigh less than 10 grams, in particular between about 0,5 and about 3 grams.

In another aspect, which is combinable with any one of the previous aspects, the patch may contain a marker to visualize the position of the patch during surgery, in particular with an X-ray, ultrasound or NMR-based means.

The disclosure further includes a kit, which is not part of the present invention, comprising a collapsible myocardial patch according to any one of the previous aspects in combination with a medical device for introduction of the patch into the pericardial sac.

In another aspect of the kit, which is combinable with the previous aspect, the introduction may be subxiphoidal. Alternatively, or additionally, the medical device may comprise a catheter with one or more means to transport adhesive material to the at least one port which is in fluid communication with at least of one of the bordering lumen and may be configured to receive the adhesive material. Additionally, the adhesive material may be a two-component adhesive. Additionally, the adhesive material may be gelling in no less than about 15 seconds, in particular between 20 and 120 seconds. Alternatively, or additionally, the adhesive material may be gelling in no more than about 150 seconds, in particular about 30 seconds. Alternatively, or additionally, the adhesive material may comprise a polyurethane-based adhesive, a PEG-based adhesive, a polyester-based adhesive, a polyvinyl-alcohol-based adhesive, a collagen-based adhesive, a gelatin-based adhesive, a chitosan-based adhesive, a dextran-based adhesive, a chondroitin-sulfate-based adhesive and/or an albumin-based adhesive, especially an albumin-glutaraldehyde-based adhesive. Alternatively, or additionally, the adhesive material may be bioerodible within a period of 1 year under in-situ conditions.

The disclosure further includes a kit (not part of the invention) comprising a collapsible myocardial patch according to any one of the previous aspects in combination with a therapeutic agent for use in treating a heart condition.

The disclosure further includes a therapeutic agent (not part of the invention) for use in treating a heart condition wherein the therapeutic agent is administered with a collapsible myocardial patch according to any one of the previous aspects.

The disclosure further describes an adhesive material for use in a surgical procedure for treating a heart condition wherein the adhesive material is used to adhere a collapsible myocardial patch according to any one of the previous aspects to an epicardial surface of the heart to administer a therapeutic agent to the heart. Additionally, the adhesive material may be a two-component adhesive. Additionally, the adhesive material may be gelling in no less than about 15 seconds, in particular between 20 and 120 seconds. Alternatively, or additionally, the adhesive material may be gelling in no more than about 150 seconds, in particular about 30 seconds. Alternatively, or additionally, the adhesive material may comprise a polyurethane-based adhesive, a PEG-based adhesive, a polyester-based adhesive, a polyvinyl-alcohol-based adhesive, a collagen-based adhesive, a gelatin-based adhesive, a chitosan-based adhesive, a dextran-based adhesive, a chondroitin-sulfate-based adhesive and/or an albumin-based adhesive, especially an albumin-glutaraldehyde-based adhesive. Alternatively, or additionally, the adhesive material may be bioerodible within a period of 1 year under in-situ conditions.

The invention further includes a kit comprising a collapsible myocardial patch according to any one of the previous aspects in combination with an adhesive material. Additionally, the adhesive material may be a two-component adhesive. Additionally, the adhesive material may be gelling in no less than about 15 seconds, in particular between 20 and 120 seconds. Alternatively, or additionally, the adhesive material may be gelling in no more than about 150 seconds, in particular about 30 seconds. Alternatively, or additionally, the adhesive material may comprise a polyurethane-based adhesive, a PEG-based adhesive, a polyester-based adhesive, a polyvinyl-alcohol-based adhesive, a collagen-based adhesive, a gelatin-based adhesive, a chitosan-based adhesive, a dextran-based adhesive, a chondroitin-sulfate-based adhesive and/or an albumin-based adhesive, especially an albumin-glutaraldehyde-based adhesive. Alternatively, or additionally, the adhesive material may be bioerodible within a period of 1 year under in-situ conditions.

### Description of the Drawings

- **Fig. 1**: shows a schematic representation of a typical interventional approach that would be used to implant the myocardial patch minimally invasively to the heart of a patient, exemplary to an epicardial surface of a heart;
- **Fig. 2a**: depicts an exemplary embodiment of the myocardial patch implanted on heart surface and its relation to the surrounding anatomical structures (pericardium not shown);
- **Fig. 2b**: depicts the exemplary embodiment of the myocardial patch of Fig. 2a and additionally shows cut-away views (A-A, B-B) made through the ventricle wall and the pericardium, respectively;
- **Fig. 3a-3b**: illustrate an exemplary embodiment of the myocardial patch prior to implantation, showing the delivery side and cover side of the myocardial patch, respectively;
- **Fig. 4a-4c**: depict midline sectioned views similar to C-C section in Fig. 3b of the myocardial patch implanted on the heart, illustrating typical embodiments of the bordering lumen(s) profile shapes;
- **Fig. 5a-5b**: depict sectioned views of the myocardial patch implanted on the heart, illustrating typical embodiments of multiple reservoir lumen configurations;
- **Fig. 6a**: depicts a sectioned view of the myocardial patch implanted on the heart with bordering lumen(s) profile shapes, but in this embodiment with only the second layer covering the bordering lumen, i.e. without a first layer covering the bordering lumen;
- **Fig. 6b**: depicts a front view of the myocardial patch of Fig. 6a, but prior to implantation, illustrating the bordering regions on the delivery side;
- **Fig. 7a-7c**: show the delivery side of the myocardial patch, schematically illustrating different embodiments of how the discharge of an adhesive from the bordering lumen(s) can be controlled;
- **Fig. 8a-8h**: show the delivery side of the myocardial patch, schematically illustrating variations of the bordering regions and related lumen(s) in the form of different lumen layouts, (lumen) port configurations and patch shapes;
- **Fig. 9**: illustrates one embodiment of the myocardial patch where the second layer is effectively replaced by the delivery device being completely removeable once the therapeutic agent has been applied and adhesion of the patch has taken place;
- **Fig. 10a-10d**: illustrate typical embodiments of how a therapeutic product like a non-gel substance carrying a therapeutic agent or a tissue engineering construct can be integrated in the myocardial patch implanted on the heart;
- **Fig. 11a-11e**: depict typical embodiments of the myocardial patch, illustrating different supply lines to the myocardial patch and corresponding connecting/severing features;
- **Fig. 12a-12d**: illustrate typical embodiments of the myocardial patch in a folded state and in an expanded state, respectively;
- **Fig. 13a-13f**: depict typical embodiments of the myocardial path and respective mechanisms that can be used to release the myocardial patch from the delivery device safely once the patch has been correctly positioned and adhered to the epicardial surface.

### Detailed Description

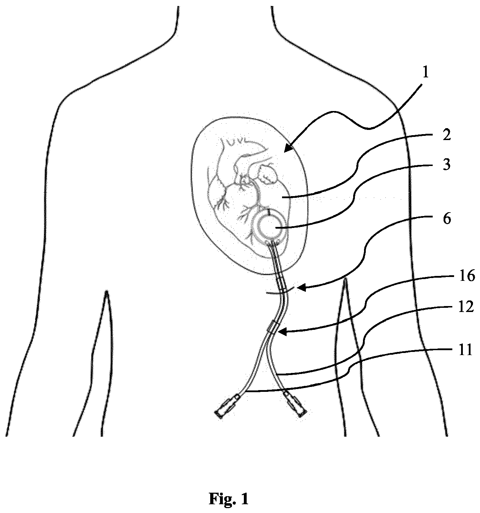

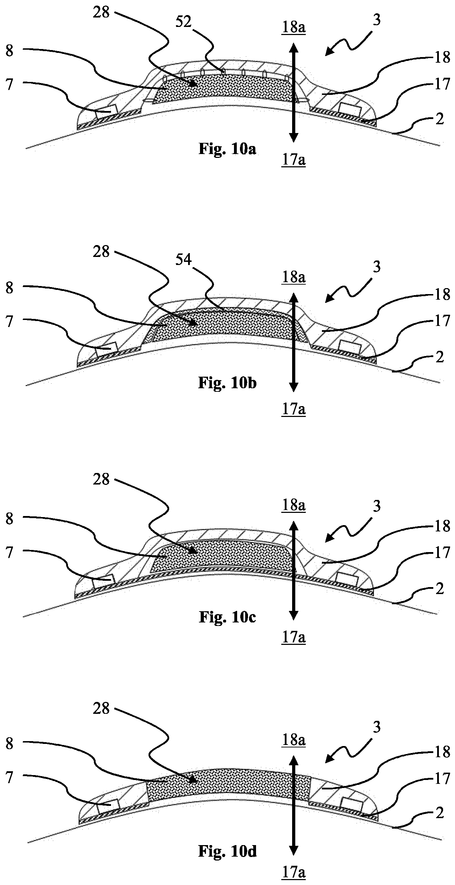

The preferred embodiment shown in **Fig. 1** depicts the myocardial patch 3, exemplary arranged on an epicardial surface 2 of the heart 1 after having been deployed through a suitable access site, like the shown subxiphoidal access route 6. At this stage, the bordering lumen 7 of the myocardial patch 3 can be filled with an appropriate adhesive to achieve adhesion between the myocardial patch 3 and the epicardial surface 2, through the bordering lumen supply line 11 connected to the bordering lumen port 9. Depending on whether the myocardial patch 3 is configured with an empty reservoir lumen 8, a therapeutic agent suspended in an appropriate carrier material 28 (see, e.g., **Fig. 10**), e.g. hydrogel, can be injected into the empty reservoir lumen 8 through the reservoir lumen supply line 12 connected to the reservoir lumen port 10. In general, the expression lumen is to be understood as a volume of a hollow space.

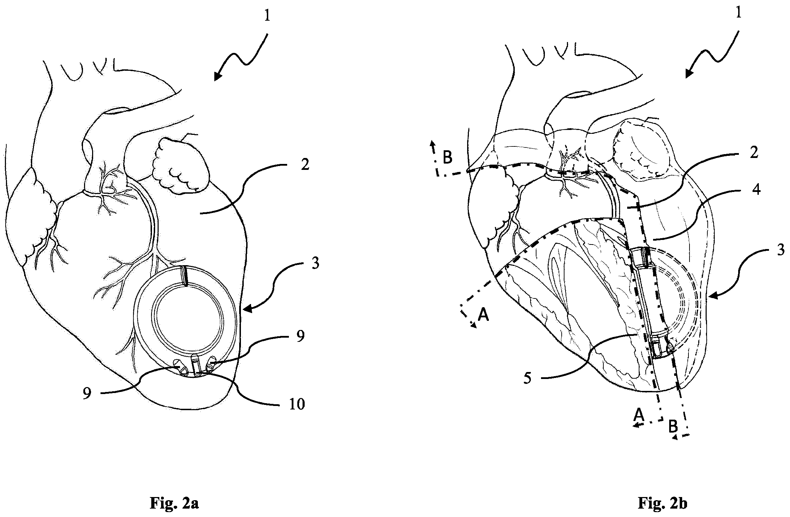

The typical placement site of the myocardial patch 3 is on the epicardial surface 2 of the heart 1, as shown in **Fig. 2a****.** In alternative embodiments, the myocardial patch could also be placed on, for instance, an inner surface of the pericardium 4, thus a surface of the pericardium 4 facing towards the heart 1 (not shown). **Fig. 2a** shows the myocardial patch 3, after adhesion to the epicardial surface 2 and after all supply lines (11, 12) and potential other connections with a delivery system have been disconnected from the respective ports (9, 10) and removed. Typically, such a myocardial patch 3 is positioned over the epicardial surface 2 of the ventricular wall 5 of the heart 1 in the pericardial space, i.e. inside the pericardium 4, as depicted in **Fig. 2b** in the sectioned (ventricular) myocardial wall 5 (Section A-A) and the pericardium 4 (Section B-B), respectively. Thus, the pericardial space is to be understood the space in between the epicardial surface 2 and the pericardium 4 (see **Fig. 2b**).

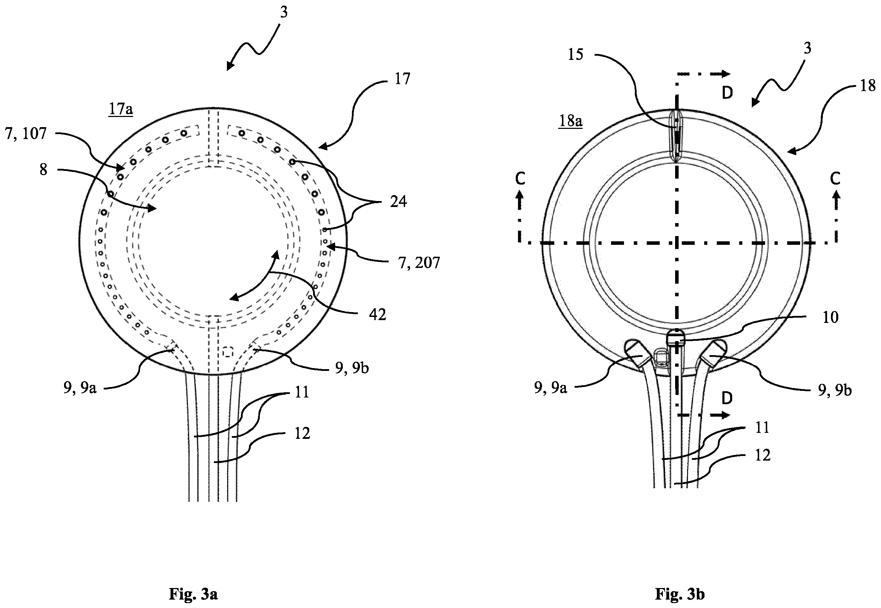

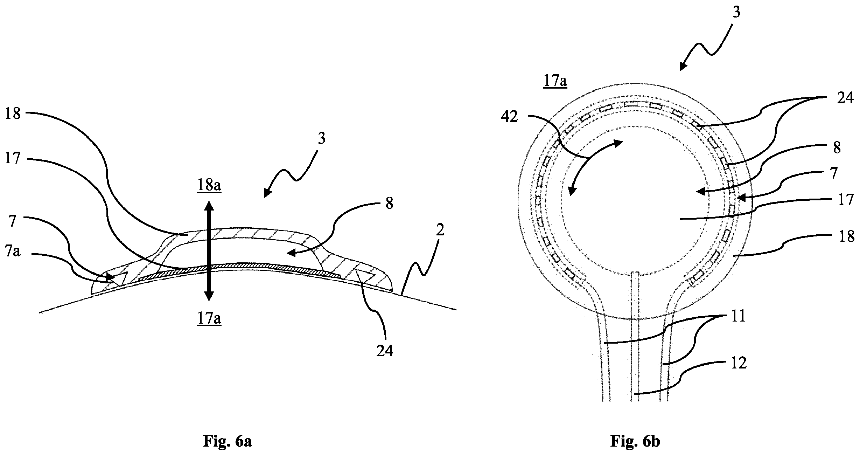

The myocardial patch 3 comprises a first layer 17 and a second layer 18, which are depicted in more detail in **Fig. 3a** and **Fig. 3b****.** The first layer 17 and the second layer 18 thereby define a delivery side 17a and a cover side 18a of the myocardial patch 3. In alternative embodiments, the myocardial patch 3 may comprise only one layer, for instance only the second layer 18, which is explained in more detail further below with regards to **Fig. 6****.** In the latter case, only the one layer may define a delivery side 17a and a cover side 18a of the myocardial patch 3.

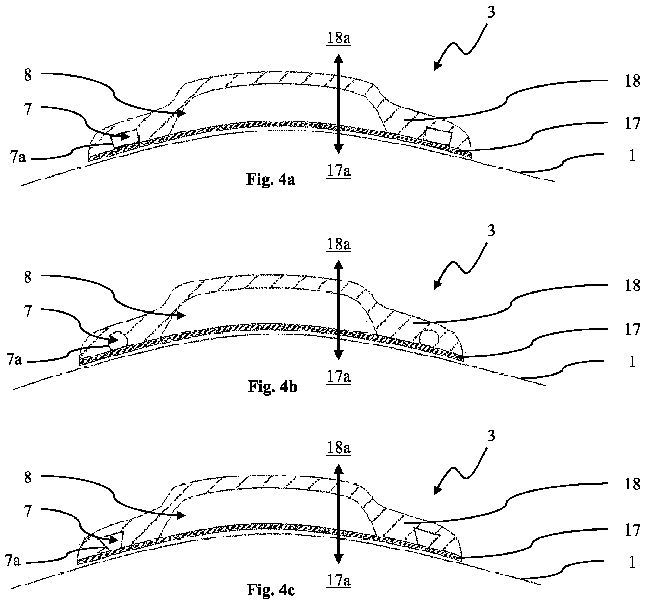

For a better understanding, **Fig. 4a** to **Fig. 4c** show section views similar to C-C section as in **Fig. 3** of the myocardial patch 3 implanted on a heart 1, including the first layer 17 and the second layer 18. The delivery side 17a of the patch 3 is to be understood as that side of the patch 3, to which the therapeutic agent is mainly delivered to the heart 1. Additionally, the delivery side 17a of the patch 3 may also be the side of the patch 3 which is arranged on a surface of a heart 1. Preferably, this surface of the heart may be an epicardial surface 2. Thus, preferably the delivery side 17a may face the epicardial surface 2 (see, e.g., **Fig. 2**). In the exemplary embodiment of **Fig. 4a** to **Fig. 4c****,** the delivery side 17a is the side of the first layer 17 and the cover side 18a is the side of the second layer 18. Thus, when the myocardial patch 3 is implanted on a heart, the first layer 17 preferably may face the epicardial surface 2 and the second layer 18 may face the inner surface of the pericardial sac 4. In alternative embodiments, especially those with only a second layer 18 and no first layer 17 (see explanations below with reference to **Fig. 6**), the delivery side 17a is on one side of the second layer 18, which faces an attachment surface of the heart 1, preferably the epicardial surface 2, and the cover side 18a is on the other side of the second layer 18, preferably the inner surface of the pericardial sac 4.

Now back to **Fig. 3a** and **Fig. 3b****,** preferably, the second layer 18 is less permeable for the therapeutic agent than the first layer 17, such that the therapeutic agent is applied at the therapy site without unnecessary loss. The first layer 17 can be permeable by nature or become permeable through biodegradation. The second layer 18 is preferably biostable for the time of the therapy. The second layer 18 can comprise an additional handling grip 15 for facilitating deployment and maneuvering during the implantation of the myocardial patch 3.

In some embodiments, the second layer 18 can be made of a biodegradable material to ensure that no or little material is left in the myocardium after the therapy is completed. When the second layer 18 is made of biodegradable material, the second layer 18 should preferably be more slowly biodegradable than the first layer 17 in order for the therapeutic agent to be released mainly to the epicardial surface 2 of the heart 1. The material need not be entirely biodegradable, however. Thus, a residue may remain from the myocardial patch 3 in a trade-off for either better therapeutic and mechanical characteristics. The first layer 17 and the second layer 18 can thereby be made of synthetic polymers.

One embodiment comprises a myocardial patch 3, wherein at least one of the first layer 17 and the second layer 18 is made of polylactic acid (also known as PLA), polyglycolic acid or copolymers thereof (also known as PLGA). When PLGA oligomers are functionalized with a double bond containing endgroups, they can be photo-crosslinked. Photopolymerization makes effective, rapid and controllable crosslinking at low temperatures possible, providing handles to control the physical properties of the networks (such as hydrophilicity and mechanical behavior) and alter degradation rates. PLA and PLGA comprise acidic degradation, which is not advantageous in every case. Further, PLA and PLGA are relatively hydrophobic. However, by using PLA or PLGA, therapeutic agents can easily be released from a reservoir lumen 8 by diffusion through the polymer and while the polymer degrades. Further, PLA and PLGA provide the ability to deliver drugs in a controlled manner over prolonged periods of time.

One embodiment comprises a myocardial patch 3, wherein at least one of the first layer 17 and the second layer 18 is made of polyesteramides. However, using polyesteramides for the myocardial patch 3 has a number of advantages. Polyesteradmides comprise safe degradation products which are not potentially harmful to the patient. Polyesteramides have protein-like physical properties and are hydrophilic, biodegradable, biocompatible and provide good tissue and blood compatibility. Furthermore, by using polyesteramides therapeutic agents can easily be released from a reservoir lumen 8 by diffusion through the polymer. In particular, polyesteramides provide zero-order kinetics drug release via surface erosion degradation. When polyesteramides are used as non-viral gene delivery vehicles, most of the DNA remains in the endocytic compartments. However, they also provide high degree plasmid DNA binding, a wide dosage range without affecting cell morphology, viability of apoptosis.

Further, polyesteramides when used as non-viral gene delivery vehicles provide cellular incorporation via endocytosis, and nearly 100 % transfection efficiency.

One embodiment comprises a myocardial patch 3, wherein at least one of the first layer 17 and the second layer 18 is made of polythioesters. For polythioesters, only a limited range of building blocks are available. However, using polythioesters for the myocardial patch 3 has a number of advantages. Polythioesters provide very flexible possibilities to tune their properties. Both linear and crosslinked polymers of polythioesters can be prepared by either thermal or photochemical polymerization. Using polythioesters, thermally sensitive compounds, for example proteins, can be processed along with photochemically sensitive compounds.

One embodiment comprises a myocardial patch 3, wherein at least one of the first layer 17 and the second layer 18 is made of a polymer composition comprising a polyurethane (PU), a polyester urethane (PEU), and/or a poly(ester urethane) urea (PEUU) elastomer. In one example, a PEULT is an elastomer made from polycaprolactone diol (MW 2000) and 1,4-diisocyanatobutane using putrescine as a diamine chain extender. Alternative to putrescine, any other useful diamine chain extender can be used. The polymer composition is preferably porous, wherein the porosity is in the range from about 60% to about 95%, for example 85%.

One embodiment comprises a myocardial patch 3, wherein at least one of the first layer 17 and the second layer 18 is made of a polymer composition comprising a polycaprolactone-b-polyethylene glycol-b-polycaprolactone triblock copolymer.

Additionally or alternatively, the first layer 17 and/or the second layer 18 can be made using at least one of the group of polyphosphazenes, polycyanaoacrylates, polyhydroxyalkanoates, polycaprolactone, polyanhydrides, polydioxanones, polyorthoesters, polypropylene fumarates, polyamido amines, collagen, fibrin, fibrogen, gelatin, cellulose, polysaccharides, starch and amylose.

In a further embodiment, the second layer 18 can comprise a material with a defined porosity that allows for ingrowth with surrounding tissue, thereby increasing at least one of the stability of the ventricular wall and the longer-term adhesion of the myocardial patch 3 to the ventricular wall. The porous material can cover the reservoir lumen 8 as well as parts of the patch adjacent to the reservoir lumen 8. When a porous material is used for the entire second layer 18, the inner surface of the bordering lumen 7 may need to be treated to allow for ease of passage of the adhesive and to avoid that the adhesive is absorbed by or infiltrates the porous material. Hereto, the inner surface of the bordering lumen 7 may be smoothed, or coated, for example.

Now with reference to **Fig. 4a**, the first layer 17 is preferably plain and slick to fit closely to the surface of the heart 1, where the myocardial patch will be attached to. Preferably, the first layer 17 is plain and slick to fit closely to the epicardial surface 2. The first layer 17 is formed to define one or more reservoir lumen 8 and one or more bordering lumen 7 together with the second layer 18. Thereby, in an area covering the reservoir lumen 8 the first layer 17 is configured to allow containment of a carrier material 28 and a therapeutic agent in the reservoir lumen 8 and to allow transport of therapeutic agent from the reservoir lumen 8 to the epicardial surface 2 over time. Transport of therapeutic agent can be achieved by diffusion through a permeable first layer 17, or via perforations (not shown) in the first layer 17 in the area defining the reservoir lumen 8, for example. In an area covering the bordering lumen 7, the first layer 17 is preferably configured to allow containment of adhesive in the bordering lumen 7 and to allow for discharge of adhesive from the bordering lumen 7. Adhesive should be discharged from the bordering lumen 7 in a distributed manner in order for the myocardial patch 3 to be evenly and strongly adhered to the epicardial surface 2. To this end, the first layer 17 can comprise openings in the area defining the bordering lumen 7 as described later.

In some embodiments, the first layer 17 covers an area of the patch including the bordering lumen 7, but excluding the reservoir lumen 8 (see, e.g., **Fig. 10a****).** The first layer 17 is then configured to allow containment of adhesive in the bordering lumen 7 and to allow for a more distributed discharge of adhesive from the bordering lumen 7. In this case, a more stable therapeutic product can be used instead of a gel carrier material 28 carrying a therapeutic agent. For example, the therapeutic product can be a non-gel substance carrying a therapeutic agent. In another example, the therapeutic product can be a 3D tissue engineering product with or without a further therapeutic agent, for example a Biomerix 3D Scaffold^{™} based product.

**Fig. 10a** shows one embodiment comprising a myocardial patch 3, wherein the first layer 17 does not cover a reservoir lumen 8 and wherein the carrier material 28 is stitched to the second layer 18. Since the carrier material 28 is not a gel material, there is no need for the first layer 17 to cover the reservoir lumen 8. Therefore, the carrier material 28 can be in direct contact with an epicardial surface 2.

**Fig. 10b** shows one embodiment comprising a myocardial patch 3, wherein the first layer 17 does not cover a reservoir lumen 8 and wherein the carrier material 28 is glued to the second layer 18. Alternatively, the bonding of shown carrier material 28 can also occur through a thermal welding process. Since the carrier material 28 is not a gel material, there is no need for the first layer 17 to cover the reservoir lumen 8. Therefore, the carrier material 28 can be in direct contact with an epicardial surface 2.

In a further embodiment, the second layer 18 itself can be impregnated with a therapeutic agent, thereby also obviating a first layer 17 covering a reservoir lumen 8.

In some embodiments, the first layer 17 covers an area of the patch including the reservoir lumen 8, but excluding the bordering lumen 7. The first layer 17 is then configured to allow containment of a carrier material 28 and a therapeutic agent in the reservoir lumen 8 and to allow transport of therapeutic agent from the reservoir lumen 8 to the epicardial surface 2 over time. In this case, the bordering lumen 7 is defined by the second layer 18 without the first layer 17.

**Fig. 6a** shows one embodiment comprising a myocardial patch 3, wherein the first layer 17 only may cover the reservoir lumen 8 in direction of the delivery side 17a. That means, the first layer 17 does not cover the bordering lumen 7 in direction of the delivery side 17a. Instead the bordering lumen 7 is defined by the second layer 18. In the example of **Fig. 6a****,** the bordering lumen 7 has a cross-section 7a in the form of a triangular prism. Therein, one edge of the triangular prism facing the epicardium 2, that is in direction of the delivery side, is sealed by the second layer 18 except for bordering lumen openings 24 configured to allow for a defined discharge of adhesive (see **Fig. 6b****).** As in the other embodiments, the reservoir lumen 8 may be covered by the second layer 18 in direction of the cover side 18a. **Fig. 6b** shows the delivery side 17a of the myocardial patch 3 with the bordering lumen 7 enclosed by the second layer 18, the sealed parts of the bordering lumen 7 and the bordering lumen openings 24 of the bordering lumen 7. Since the first layer 17 does not cover the bordering lumen 7, the first layer 17 can be better suited to allow for containment and transport of the therapeutic agent to the heart 1. It is clear that a bordering lumen 7 defined by the second layer 18 only does not need to have a triangular cross-section. The bordering lumen 7 can, for example, have a round, rectangular or any other shaped cross-section 7a (see also **Fig. 4a-4c****).** Therein, the bordering lumen 7 is sealed partially by the second layer 18, and partially the bordering lumen 7 is open for adhesive to discharge from the bordering lumen 7. The bordering lumen 7 can have any other cross-section 7a allowing for a defined discharge of adhesive via openings 24 or perforations in the second layer 18. Preferably, the openings are evenly spaced across the bordering lumen 7 on the delivery side 17a of the myocardial patch 3. As will be described, the openings 24 can also be arranged differently, for example with decreasing spacing and/or growing size in a circumferential direction 42.

In some embodiments, a first layer 17 may be entirely dispensable (not shown). A non-gel carrier material 28 with a therapeutic agent can be fixated, for example stitched or glued, to the second layer 18, or the second layer 18 itself can be impregnated with a therapeutic agent. Therefore, the reservoir lumen 8 can be open to the delivery side 17a, as described before. The bordering lumen 7 can be defined by the second layer 18 alone, with adequate openings 24 for a discharge of adhesive, as described before.

Thus, in alternative embodiments the myocardial patch 3 may comprise only one layer. This one layer may define a bordering lumen having openings and/or perforations arranged circumferentially in a peripheral region of the layer. Thereby, the one layer may further define a cavity or recess radially inside the circumferentially arranged openings of the bordering lumen, the cavity or recess having an opened side towards the delivery side of the patch which may be configured to receive non-gel carrier material 28.

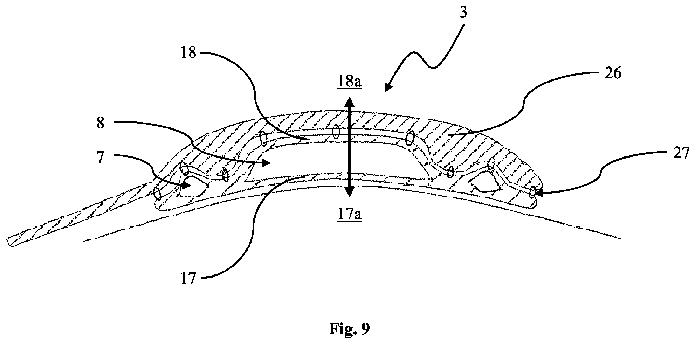

**Fig. 9** shows one embodiment comprising a myocardial patch 3, preferably in a sectioned view similar to section C-C of **Fig. 3b****,** wherein the second layer 18 may consist of the same material as the first layer 17, which is typically thinner and more permeable. Preferably, the bordering lumen 7 comprises openings (not visual in **Fig. 9**) to the delivery side 17a as described for other embodiments. Other than that, the myocardial patch 3 comprises an essentially symmetric structure, resembling a tea bag containing a carrier material 28 (not visual in **Fig. 9**) with therapeutic agent. In particular, the reservoir lumen 8 does not have a shape predefined by the second layer 18 and closed by the first layer 17. Rather, the reservoir lumen 8 has an undefined shape per se. When the reservoir lumen 8 is filled, its shape conforms with the shape of its content. Similarly, the bordering lumen 7, especially the cross-section 7a of the bordering lumen 7, does not have a shape predefined by a second layer 18 and closed by a first layer 17. Rather, the bordering lumen 7, especially the cross-section 7a of the bordering lumen 7, has an undefined shape per se. When the bordering lumen 7 is filled, its shape conforms with the shape of its content. Preferably, a delivery device 26 (depicted in a sectioned view similar to section D-D of **Fig. 3b**) is configured to impose a predefined shape onto the reservoir lumen 8 and the bordering lumen 7 during an initial filling of the reservoir lumen 8 and the bordering lumen 7. Therefore, the reservoir lumen 8 and the bordering lumen 7 can be filled without significant resistance. Therein, the delivery device 26 is releasably connected to the patch 3 using any suitable means of fixation 27, for example one of the releasable connections described herein. When the reservoir lumen 8 is filled with a gel carrier material 28, the reservoir lumen 8 takes a rounded flat shape. After the bordering lumen 7 is filled with an adhesive, the adhesive is discharged onto the epicardial surface 2 and the bordering lumen 7 is basically emptied, taking a flat shape. Where the stability of the patch needs to be upheld, further measures may be necessary to compensate for the lack of a stable second layer 18 and the lack of ingrowth. As soon as the stability of the patch is ensured, the delivery device 26 can be released from the patch. By making the second layer 18 of the same material as the first layer 17, a very thin myocardial patch 3 is possible. Thereby, fibrosis is reduced and the myocardial patch 3 can be implanted very easily. Furthermore, little foreign material needs to be introduced into the patient body, reducing the risk of an incompatibility effect like necrosis, inflammation, an immune response or an infection.

As described before, a reservoir lumen 8 is configured to allow containing a therapeutic agent and releasing it to the heart in a defined way. Partially, the first layer 17 and the second layer 18 of the myocardial patch 3 are undetachably connected to each other. In other parts, the first layer 17 and the second layer 18 are not connected to each other. The reservoir lumen 8 is defined by the first layer 17 and the second layer 18 between a joining of the first layer 17 and the second layer 18. Thereby, the first layer 17 mainly restricts the reservoir lumen 8 to a delivery side 17a and the second layer 18 mainly restricts the reservoir lumen 8 to a cover side 18a.

**Fig. 4a** shows one embodiment comprising a myocardial patch 3, wherein a reservoir lumen 8 is defined by the first layer 17 and the second layer 18 in between a joint of the first layer 17 and the second layer 18. The connection between the first layer 17 and the second layer 18 is achieved, for example, by thermal welding or by gluing.

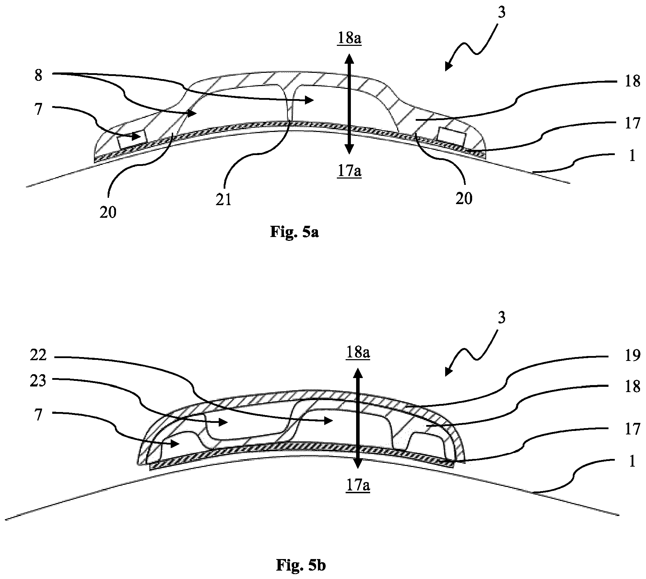

**Fig. 5a** shows one embodiment comprising a myocardial patch 3, wherein two reservoir lumens 8 are defined between a first joint 20 of the first layer 17 and the second layer 18 and a second joint 21 between the first layer 17 and the second layer 18. Thereby, the first joint 20 extends ring-like in a circumferential direction 42 (compare **Fig. 3a****)** and the second joint 21 extends line-like, for example from a proximal side of the patch 3 to a distal side of the patch 3. The connection between the first layer 17 and the second layer 18 is achieved, for example, by penetration welding or by gluing. Two reservoir lumens 8 can be used, for example, to deliver two different therapeutic agents to the heart. This can be advantageous when the therapeutic agents should not be mixed. Two reservoir lumens 8 can also be used, for example, to deliver a particular therapeutic agent at one release rate from one reservoir lumen 8 and at another release rate from another reservoir lumen 8. This can be achieved with different geometry of the reservoir lumens 8, differences in the first layer 17 and/or differing carrier material 28. Two reservoir lumens 8 can also be used when two therapeutic agents need to be contained using different carrier material 28, respectively. In alternative embodiments, myocardial patch 3 may comprise more than two reservoir lumens 8. More than two reservoir lumens 8 can be advantageous for the same reasons and achieved using the similar structures.

**Fig. 5b** shows one embodiment comprising a myocardial patch 3, wherein two reservoir lumens 8 are defined. A delivery side 17a reservoir lumen 22 is defined by the first layer 17 and the second layer 18 between a joint of the first layer 17 and the second layer 18 as described before. A cover side 18a reservoir lumen 23 is defined by the second layer 18 and a third layer 19 facing the cover side 18a between a joint of the second layer 18 and the third layer 19. The delivery side 17a reservoir lumen 22 is separated from a first heart surface, preferably the epicardial surface 2, by the first layer 17 only, which is preferably permeable to a therapeutic agent. The delivery side 17a reservoir lumen 22 is separated from a second heart surface (not shown) located on the opposing side of the first heart surface in direction of the cover side 18a, preferably the inner surface of the pericardium 4, by the second layer 18 and the third layer 19, on the other side, thereby releasing less or no therapeutic agent to the pericardium 4. The cover side 18a reservoir lumen 23 is separated from the second heart surface, preferably the inner surface of the pericardium 4, by the third layer 19 only, which is preferably permeable to a therapeutic agent. The cover side 18a reservoir lumen 23 is separated from the first heart surface, preferably the epicardial surface 2, by the second layer 18 and the first layer 17, on the other side, thereby releasing less or no therapeutic agent to the first heart surface, preferably the epicardial surface 2. Thus, a therapeutic agent only intended to be applied to a first heart surface on a delivery side 17a of the patch 3, preferably the epicardial surface 2, can be contained in the delivery side 17a reservoir lumen 22 and at the same time a therapeutic agent only intended to be applied to a second heart surface on a cover side 18a of the patch 3, preferably the inner surface of the pericardium 4 can be contained in the cover side 18a reservoir lumen 23. Thereby, the third layer 19 can be made in the same way as the first layer 17, or it can be different to better suit the second heart surface on a cover side 18a of the patch 3, preferably the inner surface of the pericardium 4, and the therapeutic agent in the cover side 18a reservoir lumen 23.

A reservoir lumen 8 can contain a gel carrier material 28 as described before. The first layer 17 and the carrier material 28 are designed based on the therapeutic agent such that the therapeutic agent can pass through the first layer 17, but the carrier material 28 in its deployed state (for example, polymerized gel or 3D tissue engineered structure fixed to the second layer 18) cannot pass through the first layer 17 when present. Thereby, passage through the first layer 17 can be based on diffusion. Passage through the first layer 17 can be based on further mechanisms alternatively or additionally.

A reservoir lumen 8 is preferably configured to allow for in-situ polymerization of a liquid gel carrier material 28. The liquid gel carrier material 28 can be easily injected into the reservoir lumen 8. Subsequently, the polymerized gel carrier material 28 is easy to be contained in the reservoir lumen 8 without leakage due to the limited permeability of the first layer 17 and second layer 18. Alternatively, the reservoir lumen 8 may be configured to allow for injection of a pre-gelled carrier material 28. A pre-gelled carrier material 28 can be flexible and having a low stiffness allowing for injection into the reservoir lumen 8 via an injection system. Nonetheless, a pre-gelled carrier material 28 can also be easily contained in a reservoir lumen 8 without leakage even when the first layer 17 covering the reservoir lumen 8 is configured to be very permeable to allow passage of a particular therapeutic agent, for example in the case that a therapeutic agent possesses a large molecular size.

Alternatively, the reservoir lumen 8 can contain a non-gel therapeutic product. In one example, a therapeutic product can be a non-gel carrier substance comprising a therapeutic agent. A non-gel carrier substance is preferably a flexible, low-stiffness solid material. In another example, a therapeutic product can be a therapeutic tissue, for example a tissue or a 3D engineered tissue product. Therein, the tissue can comprise a therapeutic agent and/or have a therapeutic effect itself. A therapeutic product can also be a combination of any of the described. Therein, any component of a therapeutic product can be fixed in the myocardial patch 3 before delivery to the heart, fixed to the myocardial patch 3 in the pericardial sac 4 or delivered to the myocardial patch 3 at the therapy site via a port to allow for fluid communication between the reservoir lumen 8 and one or more supply tubes.

In general, the expressions supply line and supply tube describe the same feature and can be used analogously. Also, the expressions pericardial sac and pericardium describe the same subject and can be used analogously.

**Fig. 10a** shows one embodiment comprising a myocardial patch 3, wherein the patch 3 comprises a reservoir lumen 8 and a non-gel therapeutic product, and wherein the therapeutic product is fixed to the second layer 18 using a suture 52. Therein, the therapeutic product is stitched to the second layer 18 loosely, somewhat decoupling the therapeutic product from movement of the myocardial patch 3 against the epicardial surface 2 which can facilitate ingrowth of the therapeutic product. Alternatively, the therapeutic product can be stitched to the second layer 18 tightly, avoiding friction from movement of the therapeutic product against the myocardial surface.

**Fig. 10b** shows one embodiment comprising a myocardial patch 3, wherein the patch comprises a reservoir lumen 8 and a non-gel therapeutic product, and wherein the therapeutic product is fixed to the second layer 18 using an adhesive 54. The therapeutic product can be glued into the myocardial patch 3 before delivery of the myocardial patch 3 to the therapy site, or glued into the myocardial patch 3 in-situ, for example using a self-adhesive second layer 18, a self-adhesive therapeutic product, or an additional adhesive delivered to the heart. The therapeutic product can also be fixed to the second layer 18 using surface treatment techniques like for example a thermal welding process.

**Fig. 10c** shows one embodiment comprising a myocardial patch 3, wherein the patch comprises a reservoir lumen 8 and a non-gel therapeutic product, and wherein the therapeutic product is held in position in the myocardial patch 3 by the first layer 17. The therapeutic product can have a spongy structure, thereby expanding to sit tightly in the reservoir lumen 8 defined by the first layer 17 and the second layer 18s. The therapeutic product can, additionally or alternatively, be pressed firmly against the first layer 17 by the second layer 18. Additionally, or alternatively, the therapeutic product can be held in position along its circumference by the second layer 18. As will be described in the following embodiment, this can even replace the fixture by the first layer 17 entirely.

**Fig. 10d** shows one embodiment comprising a myocardial patch 3, wherein the patch comprises a reservoir lumen 8 and a non-gel therapeutic product, and wherein the therapeutic product is held in position in the myocardial patch 3 along its circumference by the second layer 18. The therapeutic product can be configured to be flexible enough to be held in place by the second layer 18 pressing against the therapeutic product along its circumference. Additionally or alternatively, the therapeutic product and the second layer 18 can be shaped to inter-lock for the therapeutic product to be fixed against the second layer 18. The second layer 18 does not cover the therapeutic product to the cover side 18a. However, alternatively, the second layer 18 can cover the therapeutic product to the cover side 18a. In that case, the second layer 18 can additionally press the therapeutic product firmly against a surface of the heart 1 on the delivery side 17a of the patch 3, preferably against the epicardial surface 2.

As described before, a bordering lumen 7 is configured to allow containment of adhesive in the bordering lumen 7 and to allow for a discharge of adhesive from the bordering lumen 7. Partially, the first layer 17 and the second layer 18 of the myocardial patch 3 are undetachably connected to each other. In other parts, the first layer 17 and the second layer 18 are not connected to each other. The bordering lumen 7 is defined by the first layer 17 and the second layer 18 with a joining of the first layer 17 and the second layer 18 in between. In some embodiments, the at least one bordering lumen 7 may be located in a peripheral region of the patch 3. The expression peripheral region is to be understood as an outer region of the patch in a direction which is orthogonal to a direction from the delivery side 17a to the cover side 18a. For instance, when regarding the patch 3 of **Fig. 3a****,** peripheral would mean in an outer region in a radial direction and/or close to the outer circumference of the patch 3.

**Fig. 3b** shows one embodiment comprising a myocardial patch 3, wherein the patch comprises a bordering lumen 7 defined by the first layer 17 and the second layer 18. The bordering lumen 7 is in fluid communication with two bordering lumen ports 9. The two bordering lumen ports 9 are each releasably connected to an adhesive supply tube 11 on its other end. That is, a bordering lumen port 9 may comprise a first end via which the bordering lumen port 9 is in fluid communication with the bordering lumen 7, and a second end which is releasably connectable to a supply tube 11. Thus, the bordering lumen port 9 may be fluidly communicatable with a supply tube 11 via the second end, when connected to the supply tube 11. Via the adhesive supply tubes 11, the patch 3 can be supplied with adhesive in vivo, when the myocardial patch 3 is already placed at the therapy site on the heart 1. Supplying the adhesive to the patch 3 only after delivering the patch 3 also allows using more reactive two component adhesives which provide a stronger bond to the heart and allow transport of the adhesive over a certain conduit length in its temporary pre-polymerized state, thereby enabling extracorporeal administration of the adhesive. Furthermore, the patch 3 can be smaller when it is delivered to the heart, thereby allowing for a less invasive provision of the patch.

In alternative embodiments, the bordering lumen 7 may be in fluid communication with only one or more than two bordering lumen ports 7. Thus, the myocardial patch 3 may comprise at least one bordering lumen port 9. Analogously, the one or more than two bordering lumen ports 7 each may be releasably connected to an adhesive supply tube 11 on its other end.

The bordering lumen 7 may be one-pieced, for instance, may circumferentially 42 extend from a first port to a second port without gap. In other embodiments, such as that one shown in **Fig. 3a**, the bordering lumen 7 may be two-pieced. For instance, a first bordering lumen section 107 may extend from a first port 9a in a first circumferential direction 42 and a second bordering lumen section 207 may extend from a second port 9b in a second circumferential direction 42, whereby the second circumferential direction 42 is opposing to the first circumferential direction 42. Thereby, the first bordering lumen section 107 and the second bordering lumen section 207 are not in fluid communication. This may advantageously improve the delivery of adhesive.

**Fig. 3a** shows one embodiment comprising a myocardial patch 3, wherein the patch comprises a bordering lumen 7 defined by the first layer 17 and the second layer 18. The bordering lumen 7 further comprises openings 24 on the delivery side 17a for adhesive to discharge from. The openings 24 are configured to contain an adhesive for the most part initially and only discharge a substantial amount of adhesive when sufficient pressure is applied. The size of the openings 24 may increase along the bordering lumen 7, the further away from the bordering lumen port 9. In other words, the size of the openings 24 may increase along a circumferential direction 42 starting from the bordering lumen port 9. Small openings 24 in relation to the viscosity of the used adhesive allow for the adhesive to initially fill the bordering lumen 7 without seeping through the openings 24. Only when the bordering lumen 7 is filled with adhesive and further pressure is applied, the adhesive discharges through the openings 24 on the delivery side 17a, preferably onto the epicardial surface 2.

**Fig. 4a** shows one embodiment comprising a myocardial patch 3, wherein a bordering lumen 7 is defined by the first layer 17 and the second layer 18 between a joining of the first layer 17 and the second layer 18. The connection between the first layer 17 and the second layer 18 is achieved, for example, by thermal welding or by gluing. The form of the bordering lumen 7 is mainly defined by the second layer 18, which comprises a channel with a rectangular cross-section opened to the delivery side 17a of the second layer 18. The channel may extend in the second layer 18 along a circumferential direction 42 as described above with reference to **Fig. 3a****.** The first layer 17 is plain and may cover the bordering lumen 7 to the delivery side 17a. The rectangular cross-section 7a of the bordering lumen 7 facilitates production with common tools, for example common cutting tools or molds. Further, a bordering lumen 7 with a rectangular cross-section 7a is suitable for evenly and predictably releasing adhesive from the bordering lumen 7 to the attachment site, e.g. the inner surface of the pericardium 4 or, preferably the epicardial surface 2.

**Fig. 4b** shows a similar embodiment comprising a myocardial patch 3, wherein a bordering lumen 7 is defined by the first layer 17 and the second layer 18 between a joining of the first layer 17 and the second layer 18. The form of the bordering lumen 7 is also mainly defined by the second layer 18, with the first layer 17 being plain and covering the bordering lumen 7 to the delivery side 17a. The second layer 18 comprises a channel with a truncated round cross-section 7a on its delivery side 17a. The channel may extend in the second layer 18 along a circumferential direction 42 as described above with reference to **Fig. 3a****.** The round cross-section 7a of the bordering lumen 7 facilitates transport of adhesive through the bordering lumen 7. This way, less pressure is needed to fill the bordering lumen 7 with adhesive, or a more viscous adhesive can be used. Furthermore, less adhesive volume remains in the bordering lumen 7 compared to a rectangular cross-section 7a shaped bordering lumen 7. A bordering lumen 7 with a round cross-section 7a can also be easily produced using common tools.

**Fig. 4c** shows a similar embodiment comprising a myocardial patch 3, wherein a bordering lumen 7 is defined by the first layer 17 and the second layer 18 between a joint of the first layer 17 and the second layer 18. The form of the bordering lumen 7 is also mainly defined by the second layer 18, with the first layer 17 being plain and covering the bordering lumen 7 to the delivery side 17a. The second layer 18 comprises a channel with a truncated triangular cross-section 7a on its delivery side 17a. The channel may extend in the second layer 18 along a circumferential direction 42 as described above with reference to **Fig. 3a****.** The triangular cross-section 7a increases the stability of the second layer 18 around the bordering lumen 7 and decreases the area that needs to be covered by the first layer 17 in relation to the bordering lumen 7 cross-sectional area. For example, the first layer 17 can therefore be designed weaker, or the bordering lumen 7 can be designed larger.

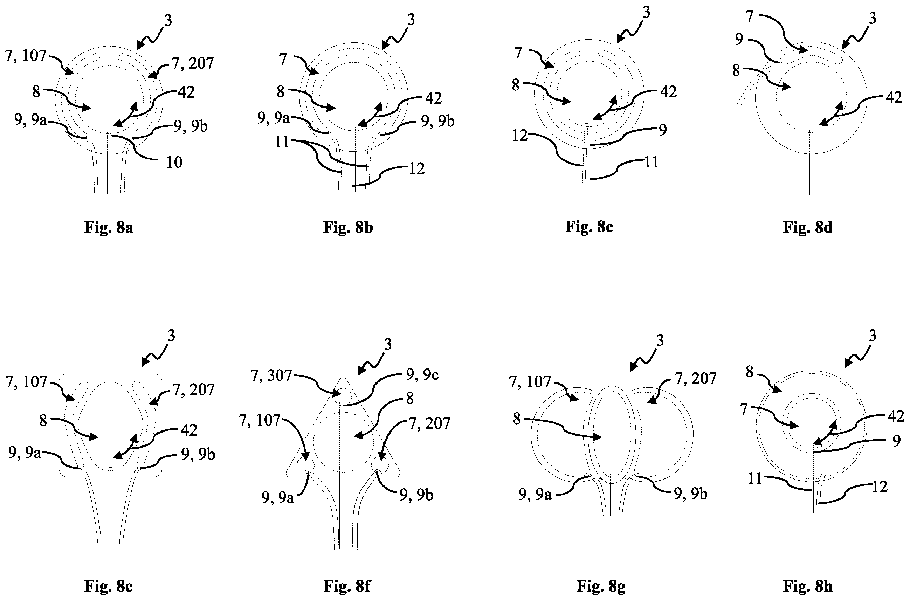

**Fig. 8a** shows one embodiment comprising a myocardial patch 3, wherein two distinct bordering lumens, a first bordering lumen section 107 and a second bordering lumen section 207 are defined by the first layer 17 and the second layer 18. The two bordering lumen sections (107, 207) have the shape of two concentric half-rings extending in a circumferential direction 42. Therefore, an element of redundancy is provided, since the adhesive from the first bordering lumen section 107 can still ensure the patch 3 is adhered to the heart in the case that the adhesion based on the second bordering lumen section 207 fails.

**Fig. 8b** shows one embodiment comprising a myocardial patch 3. The myocardial patch 3 may comprise one bordering lumen 7 which is defined by the first layer 17 and the second layer 18. The bordering lumen 7 has the shape of a ring extending in a circumferential direction 42 around a reservoir lumen 8 interrupted on the proximal side.

The expression "proximal" describes an area which is closer to an operator and/or a supply apparatus which may feed the patch 3 with fluids, for instance adhesives and/or a therapeutic agent. The counterpart of the expression proximal is "distal" which describes an area closer to the heart at the area of treatment. Similar explanation accounts for the respective directions.

That means, for instance regarding **Fig. 8b**, the bordering lumen supply lines 11 are proximal of the respective bordering lumen ports (9a, 9b). On the other hand, the bordering lumen ports (9a, 9b) are arranged in a distal direction from the respective bordering lumen supply line 11. The bordering lumen 7 in **Fig. 8a** for instance, has two interruptions thereby creating two bordering lumens (107, 207). A first interruption located on a proximal side of the patch, so a side which is closer to a supply source when going through the lumens and supply lines and in this case the side of the patch 3 where the ports are located, so the bottom of **Fig. 8a****.** A second interruption is located on a distal side of the patch, that is, the opposing side in a distal direction, so at the top of **Fig. 8a****.**

Now back to **Fig. 8b****,** the bordering lumen 7 comprises a bordering lumen port 9 at each of its ends, where the ring is interrupted. That is, port 9a and port 9b. The bordering lumen 7 is in fluid communication with each of the ports (9a, 9b) at the same time. Therefore, the bordering lumen 7 can be filled from each of the ports (9a, 9b) or both ports (9a, 9b) at the same time, allowing for a more complete initial filling of the bordering lumen 7. Further, the two ports (9a, 9b) provide an element of redundancy since, in principal, the ring can be filled via one port (either 9a or 9b) only if the filling via the other port fails.

**Fig. 8c** shows one embodiment comprising a myocardial patch 3, wherein a bordering lumen 7 is defined by the first layer 17 and the second layer 18. The bordering lumen 7 has the shape of a ring around a reservoir lumen 8. The ring is interrupted on the distal side of the patch. In an alternative embodiment, the ring may be uninterrupted. Further, the bordering lumen 7 comprises a port 9 tangential to the ring on the proximal side. The bordering lumen 7 is in fluid communication with the port 9, wherein, for a fluid coming from the port 9, there are two paths through the bordering lumen 7. One path extending clockwise in a circumferential direction 42, the other patch extending counter clockwise in a circumferential direction 42. When an adhesive is filled into the bordering lumen 7 via the port 9, the adhesive is transported through the bordering lumen 7 according to the path of least resistance. Therefore, the bordering lumen 7 can be filled quickly and with one bordering lumen supply tube 11 only.

**Fig. 8d** shows one embodiment comprising a myocardial patch 3, wherein a bordering lumen 7 is defined by the first layer 17 and the second layer 18. The bordering lumen 7 has the shape of a stripe close to the reservoir lumen 8. Preferably, the shape of the bordering lumen 7 bends around the reservoir lumen 8 in a circumferential direction 42. Furthermore, the bordering lumen 7 comprises a port 9 at one end of the bordering lumen 7 in fluid communication with the bordering lumen 7. In the exemplary embodiment of **Fig. 8d** the port 9 is arranged at the end of the bordering lumen 7 in a counter clockwise circumferential direction 42. In other embodiments, the port 9 may also be arranged at the other end of the bordering lumen 7 in a clockwise circumferential direction 42. In other embodiments, the bordering lumen may comprise two ports 9, one at each end of the bordering lumen 7. Since the bordering lumen 7 does not extend over the entire circumference of the patch 3, movement of the heart surface (e.g. epicardial surface 2 or inner surface of the pericardial sac 4), where the patch 3 is attached to when implanted on a heart 1, relative to the patch 3 is not transferred to the patch 3. Therefore, load on the patch 3 is reduced when there is a lot of movement of the heart surface (e.g. epicardial surface 2 or inner surface of the pericardial sac 4) relative to the patch 3.

**Fig. 8e** shows a similar embodiment comprising a myocardial patch 3, wherein two bordering lumens 7, a first bordering lumen section 107 and a second bordering lumen section 207 are defined by the first layer 17 and the second layer 18. The bordering lumens 7 have the shape of stripes along the reservoir lumen 8. Preferably, the shape of the bordering lumen 7 bends around the reservoir lumen 8. Furthermore, the bordering lumens (107, 207) comprise a port (9a, 9b) each, wherein each port (9a, 9b) is in fluid communication with the respective bordering lumen (107, 207). Since the bordering lumens (107, 207) do not extend over the entire circumference of the patch 3, movement of the heart surface (e.g. epicardial surface 2 or inner surface of the pericardial sac 4), where the patch 3 is attached to when implanted on a heart 1, relative to the patch 3 is not transferred to the patch 3. Therefore, load on the patch 3 is reduced when there is a lot of movement of the heart surface (e.g. epicardial surface 2 or inner surface of the pericardial sac 4) relative to the patch 3. Furthermore, an element of redundancy is introduced, since the adhesive based on one bordering lumen (107, 207) can still keep the patch 3 in place on the heart 1 in case the adhesive based on the other bordering lumen (107, 207) fails. Depending on the movement and the geometry of the of the heart surface (e.g. epicardial surface 2 or inner surface of the pericardial sac 4) relative to the patch 3, a different number of bordering lumens 7 can be used.

**Fig. 8f** shows one embodiment comprising a myocardial patch 3, wherein three bordering lumens 7, a first bordering lumen section 107, a second bordering lumen section 207 and a third bordering lumen section 307 are defined by the first layer 17 and the second layer 18. The bordering lumens (7; 107, 207, 307) may have the shape of dots or circles. The bordering lumens (7; 107, 207, 307) may comprise a port (9a, 9b, 9c) each, wherein each port (9a, 9b, 9c) is in fluid communication with the respective bordering lumen (7; 107, 207, 307). Since the bordering lumens (7; 107, 207, 307) only fixate the patch to the heart point-like surfaces, movement of the heart surface (e.g. epicardial surface 2 or inner surface of the pericardial sac 4), where the patch 3 is attached to when implanted on a heart 1, relative to the patch 3 is not blocked by an extended adhering area. Therefore, load on the adhering area is reduced when there is a lot of movement of the heart surface (e.g. epicardial surface 2 or inner surface of the pericardial sac 4) relative to the patch 3. Depending on the movement and the geometry of the heart surface (e.g. epicardial surface 2 or inner surface of the pericardial sac 4) relative to the patch 3, a different number of bordering lumens 7 can be used.

**Fig. 8g** shows a similar embodiment comprising a myocardial patch 3, wherein two bordering lumens 7, a first bordering lumen section 107 and a second bordering lumen section 207 are defined by the first layer 17 and the second layer 18. The bordering lumens 7 have the shape of half-circles surrounding the oval reservoir lumen 8 on two sides. Thus, in this exemplary embodiment, the bordering lumens (107, 207) may surround the reservoir lumen on two opposing sides of the patch, which are in an orthogonal direction to the direction from proximal to distal. Preferably, the shape of the bordering lumen (107, 207) slightly bends around the reservoir lumen 8. Furthermore, the bordering lumens 7 comprise a port (9a, 9b) each, wherein each port (9a, 9b) is in fluid communication with the respective bordering lumen (107, 207).

**Fig. 8h** shows one embodiment comprising a myocardial patch 3, wherein a bordering lumen 7 is defined by the first layer 17 and the second layer 18. Thereby, the bordering lumen 7 has the shape of a circular disk in the center of the patch. The bordering lumen 7 comprises a port 9 in fluid communication with the bordering lumen 7. The patch 9 further comprises a reservoir lumen 8 in the shape of a ring concentric to the circular disk. The reservoir lumen 8 is extending in a circumferential direction 42 around the centered bordering lumen 7. Such embodiment is particularly advantageous for use in an infarct treatment. In the center of an infarct, the myocardial cells are dead and cannot be treated. However, in an infarct border zone surrounding the center of the infarct, there is an increasing chance of recovery for the myocardial cells. Therefore, by arranging a reservoir lumen 8 with a therapeutic agent in the shape of a ring covering the infarct border zone, the patch is most effective. By arranging a border lumen with adhesive in the shape of a disk enclosed by the reservoir lumen 8, the patch 3 can be very small, allowing for less invasive operations.

Any of the described structures could be combined in a suitable manner to achieve more redundant and stronger fixation of the patch 3 to the heart 1 and to adapt better to the shape of a particular heart. Any bordering lumen 7 is in fluid communication with at least one port for filling the bordering lumen 7 with adhesive. Therein, a bordering lumen 7 may be filled via another bordering lumen 7 it is in fluid communication with. When filling the bordering lumen 7, the adhesive discharges from the bordering lumen 7 through openings 24 in the area of the first layer 17 covering the bordering lumen 7. Thus, the adhesive discharges from the bordering lumen 7 through openings 24 in the area of the first layer 17 on a delivery side 17a. Any of the described structures may also comprise only one layer, e.g. the second layer 18 which defines the respective bordering lumen(s) 7 and reservoir lumen 8 as described with reference to **Fig. 6****.** Thus, in embodiments with only a second layer 18 in the area of the bordering lumen 7, when filling the bordering lumen 7, the adhesive discharges from the bordering lumen 7 through openings 24 in the area of the second layer 18 on a delivery side 17a.

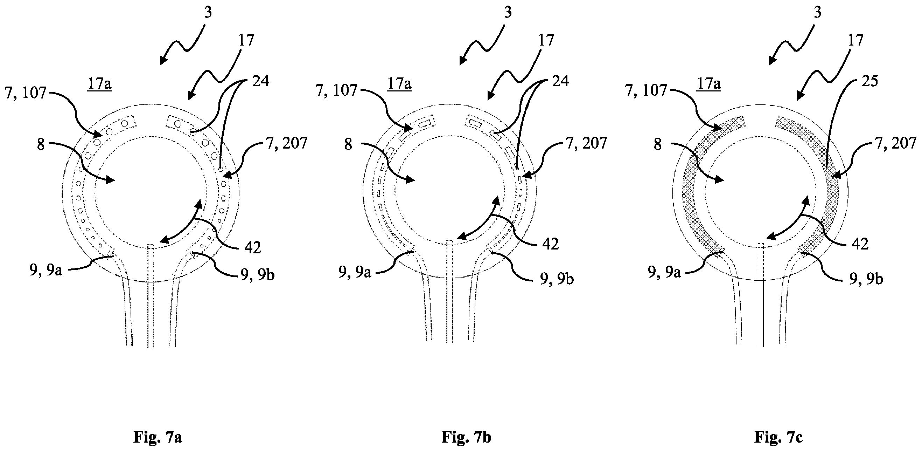

Now with reference to **Fig. 7a**, further properties of the openings 24 in the first layer 17 for adhesive discharge from the bordering lumen 7 are described. The bordering lumen 7 does not comprise openings 24 for adhesive discharge at a proximal end of the bordering lumen 7. Preferably, the first 3 millimeters to 20 millimeters of the bordering lumen 7 do not comprise openings for adhesive discharge so a stream of adhesive can build up in the initial filling without already discharging from the first openings 24. The size of the openings 24 increases from the proximal openings 24 to the distal openings 24. That means the size of the openings 24 increases along the bordering lumen 7 in a circumferential direction 42. This is particularly advantageous where the adhesive is liquid initially and increases its viscosity quickly while being filled into the bordering lumen 7. For example, an adhesive can initially have a viscosity close to that of water. After approximately 30 seconds, gelation by cross-linking can commence, increasing the viscosity of the adhesive. After approximately 120 seconds cross-linking is finished, such that the adhesive is highly viscous and cannot pass any openings anymore. Using such an adhesive, when the initial filling of the bordering lumen 7 has begun, the adhesive in its temporarily liquid phase does not seep out the first openings too much since these are small. When the distal parts of the bordering lumen 7 are filled, the then more viscous adhesive can still discharge through the bigger openings towards the distal end of the bordering lumen 7. By the time the adhesive is fully cross-linked, the filling and discharge process is finished. The exact dimensions of the openings and the variations therein depend on the injection rate, the gelation kinetics and the dimensions of the bordering lumen 7. The openings 24 are of a round shape for easy production and even discharge of adhesive.

**Fig. 7b** shows an embodiment comprising a myocardial patch 3, wherein the openings in the first layer 17 covering the bordering lumen 7 are of rectangular shape. Rectangular openings 24 can be easier to produce and can facilitate the discharge of adhesive from the bordering lumen 7. Different shapes of the openings are also possible, for example triangular shapes, diamond shapes or star shapes.

**Fig. 7c** shows an embodiment comprising a myocardial patch 3, wherein the first layer 17 covering the bordering lumen 7 comprises no distinct openings 24. Instead, a mesh layer covers the bordering lumen 7. The mesh layer can be achieved by attaching a mesh structure 25 onto an opening in the first layer 17, for example a metal mesh structure 25, a synthetic material mesh structure 25 or a textile mesh structure 25. The mesh layer can also be achieved by applying a mesh structure 25 to the first layer 17 itself, for example by cutting or 3d-engineering. Other mesh-like structures can include, for example woven or braided structures. A mesh layer has the advantage of a more even and areal distribution of adhesive on the heart surface (e.g. epicardial surface 2 or inner surface of the pericardial sac 4), where the patch 3 is attached to when implanted on a heart 1.

In another embodiment, the first layer 17 comprises openings 24 for adhesive discharge which are configured to prevent adhesive from flowing towards an area of the first layer 17 covering a reservoir lumen 8. Openings 24 can be positioned at an end of the bordering lumen 7 opposite of the reservoir lumen 8. Instead or additionally, openings 24 can be angled away from the part of the first layer 17 covering the reservoir lumen 8.

The previously described variants of openings for adhesive discharge in the first layer 17 can be used in the second layer 18 in the case where the bordering lumen 7 is defined by the second layer 18 alone, without a first layer 17 covering the bordering lumen 7.

One embodiment comprises a myocardial patch 3 fabricated into a flat shape. The myocardial patch 3 can comprise, for example, a flat first layer 17. The patch 3 can be configured to adapt to basically any therapy site and to be usable for most areas along the heart 1, in particular when the patch 3 size is small in relation to the heart 1. To this end, the patch 3 can be flexible and/or even allow a degree of movement of its layers against each other. The myocardial patch 3 can further be configured to take the shape it adapts to when adhered to the therapy site over time. The myocardial patch 3 can be easier to produce and more flexible to use. In particular, a therapy site can be varied during an operation with a single prepared myocardial patch 3.

One embodiment comprises a myocardial patch 3 prefabricated into a 3D shaped structure to conform a pre-defined range of therapy sites. In one example, a myocardial patch 3 can comprise a shape designed to fit closely to a therapy site along the circumference of a heart 1, comprising slight curvatures along a first direction and being basically flat along a second direction orthogonal to the first direction. In another example, a myocardial patch 3 can comprise a shape designed to fit closely to a therapy site at the apex of a heart, comprising high curvatures in all directions. A myocardial patch 3 can be designed to fit to a class of therapy sites for a heart 1 of a pre-defined size, or to fit to a class of therapy sites for any heart. The myocardial patch 3 can be allowed to be stiffer and still sit more tightly on the surface of the specific heart 1. Larger myocardial patches 3 in relation to the heart size can be used, allowing for larger adhesive areas and therefore better fixation of the patch and/or larger therapeutic areas and/or additional functions of the myocardial patch 3. At the same time, a large number of patches can be produced at a lower cost. A slight variation of the therapy site during operation is still possible. Further, therapy sites can be accessible that cannot be served using a flat myocardial patch at all.

One embodiment comprises a myocardial patch prefabricated into a 3D shaped structure to conform a pre-defined therapy site on a specific heart. The patch can be designed to mimic the contours of the epicardial surface of a specific patient. The patient heart can be analyzed using a medical imaging method, for example a computed tomographic method, to get a 3D model of the shape of the patient heart. Subsequently, the myocardial patch can then be designed to conform a specific therapy site on the heart and be fabricated to fit closely to the epicardial surface at the therapy site. In the process, a physical model of the complete heart or of the epicardial surface at the therapy site can be used for fabricating and/or testing the myocardial patch. The myocardial patch can be allowed to be stiffer and still sit perfectly close on the patient heart. Larger myocardial patches in relation to the heart size can be used, allowing for larger adhesive areas and therefore better fixation of the patch and/or larger therapeutic areas and/or additional functions of the myocardial patch. Anomalies of a heart can be accounted for, allowing for better use of a myocardial patch when patches of predefined shapes do not fit.

One embodiment comprises a myocardial patch 3, wherein at least a part of the second layer 18 induces ingrowth of the myocardial patch 3. Alternatively, the second layer 18 comprises a surface area to induce tissue over-growth, for example by means of a coating or a surface modification, on the cover side 18a of the patch 3, preferably the side of the second layer facing the pericardium. By inducing ingrowth of the myocardial patch, the patch is more stable and more durably fixed to the epicardial surface. Further, the growth of tissue over the myocardial patch 3 can itself be advantageous to mechanically stabilize the pathologically disadvantaged patient heart. By using a partial surface area inducing tissue over-growth, the ingrowth can the controlled more precisely by varying the coating or surface modification over the second layer 18.

One embodiment comprises a myocardial patch 3, wherein at least parts of the myocardial patch 3 are biodegradable within two years. Biodegradable in this context does not have to mean that the patch 3 is biodegraded by 100% within after the predetermined time frame (in this case two years) at the implanted therapy site in-vivo. Biodegradable rather means that when conducting an ex-vivo test with specific test conditions (e.g. a certain temperature and pH level), for instance using a saline solution, the myocardial patch 3 may be degraded within two years. Considering the fact, that the in-vivo conditions at each therapy site may vary from patient to patient, the biodegradation may occur faster or slower and thus, the patch 3 may be biodegraded by 100% depending on the therapy site conditions within sooner or later than two years. Preferably, the myocardial patch 3 is biodegradable between two weeks and six months, but not before the therapy is finished. A biodegradable myocardial patch 3 does not need to be removed from the therapy site after a therapy and does not reside on the heart surface forever after the therapy. Rather, the patch is decomposed by the patient organism over time. Therein, the materials, in particular, the polymer compositions, used for the structure of the myocardial patch 3, the carrier material 28, the adhesive used via the bordering lumen 7, and/or any further components used with the myocardial patch are biodegradable. However, some residue of the myocardial patch may be left in the patient body or even at the therapy site in the long-term. Therefore, the materials used for the patch or degradation products thereof may not cause a substantial adverse reaction or substantial harm to cells and tissues in the body like necrosis, chronic inflammation, a chronic immune response or an infection resulting in harm to tissues from the implanted patch. To this end, preferably only cytocompatible polymer compositions are used, i.e. the polymer compositions and degradation products thereof can sustain a population of cells, are not cytotoxic, and are not carcinogenic. When placed in a human myocardial cell culture a cytocompatible polymer does not adversely affect the viability, growth, adhesion, and number of cells and is substantially non-toxic and non-carcinogenic in a patient within useful, practical and acceptable tolerances.

In general, a myocardial patch described herein may be substantially planar having much greater dimension in two dimensions and a substantially smaller dimension in a third, comparable to bandages, gauze, and other substantially flexible, flat items. In one example, the myocardial patch may be as thick as the heart wall of a patient. In one embodiment, the patch is substantially planar having a large size in two dimensions and a substantially smaller size in a third dimension. Besides flat, planar patches, the myocardial patch can also have three-dimensional shapes useful to fit specific therapy sites on the heart. Further, the myocardial patch can comprise a three-dimensional structure useful for the treatment of specific deficiencies, for example to induce fibrosis in a particular shape. The myocardial patch comprises a thickness ranging from about 50 µm to about 5 mm, preferably between 500 µm and 1.5 mm.

One embodiment comprises a myocardial patch, wherein at least one of the first layer or the second layer is porous. A porous material comprises a large portion of pores (e.g., spaces, gaps, holes, or openings), making up for example for at least 60% of the volume of the layer not connected to the second layer, or for example for at least 90% of the volume of the layer not connected to the second layer, or any increment between those. A porous material can have, for example, a mesh structure, a textile structure, or a sponge structure. The pores of the porous material can be filled with any of water, air, pericardial fluid, a specifically chosen or specifically designed fluid, or anything else. Porous materials are preferably produced using electrospinning. Alternatively, porous material can be produced using, for example, solvent casting, salt leaching, or thermally induced phase separation, or any other common process used to produce porous material. A porous first layer can be advantageous, amongst others, to the release of therapeutic agent, to the distribution of adhesive, the ingrowth of the patch, the flexibility of the myocardial patch, provide partial self-adhesion properties through capillary effects and easy delivery of the patch to the therapy site. A porous second layer can be advantageous, amongst others, the ingrowth of the patch, the flexibility of the myocardial patch, and easy delivery of the patch to the therapy site.

One embodiment comprises a myocardial patch made of elastomeric polymer compositions. Preferably, highly distensible polymer compositions are used. Polymers with a breaking strain ranging from about 100% to about 900%, preferably between 325% and 600%, are used in the elastomeric polymer composition. Alternatively, the breaking strain of a polymer is between 50% and 100%. Further, polymers with a tensile strength ranging from about 10 kPa to about 30 MPa, preferably between 6 MPa and 20 MPa, are used in the elastomeric polymer composition. Further, polymers with an initial modulus ranging from about 10 kPa to about 100 MPa, for example between 20 MPa and 60 MPa, are used.

One embodiment comprises a myocardial patch and a delivery device, wherein the delivery device comprises a marker. In one example, a marker is a material or structure resonant to ultra-sound, thereby revealing the position of the delivery device using a sonographic unit. In another example, a marker is a material or structure absorbent of x-radiation, thereby revealing the position of the delivery device using an x-ray unit. Similarly, a marker can be a material or structure revealing the position of the delivery device in an NMR or another medical imaging method.

One embodiment comprises a myocardial patch, wherein the myocardial patch comprises a marker. In one example, a marker is a material or structure resonant to ultra-sound, thereby revealing the position of the myocardial patch using a sonographic unit. In another example, a marker is a material or structure absorbent of x-radiation, thereby revealing the position of the myocardial patch using an x-ray unit. Similarly, a marker can be a material or structure revealing the position of the myocardial patch in an NMR or another medical imaging method. A marker comprised by the myocardial patch is preferably resorbing, so it does not remain in the body of the patient possibly being harmful and/or interfering with medical imaging unrelated to the implantation of the myocardial patch.

One embodiment comprises a myocardial patch and a protective film covering the first layer of the myocardial patch. Therein, the protective film can be a part of a delivery device, or a separate film connected to the myocardial patch to be removed in situ by a delivery device. The protective film covers the first layer before insertion into the patient body and is removed only shortly before filling the bordering lumen with adhesive. By covering the first layer with the protective film, bodily fluid is prevented from filling the reservoir lumen or the bordering lumen. Further, the protective film secures the first layer against tearing during the implantation.

One embodiment comprises a myocardial patch, wherein the myocardial patch itself releases the primary therapeutic agent or a secondary drug. In one example, when the materials, in particular the polymer compositions, used for the structure of the myocardial patch, the carrier material, the adhesive used via the bordering lumen, and/or any further components used with the myocardial patch, decompose in the patient body over time, at least one of the degradation products may provide a therapeutic benefit. In one specific example, the individual building block of the polymers may be chosen such that the building blocks provide a therapeutic benefit when released in situ. In yet another specific example, a polymer can comprise putrescine building blocks, which when released through degradation causes cell growth and cell differentiation. In another example, a therapeutic agent is introduced into the myocardial patch in the process of production. In a specific example, during the solvent casting process, the therapeutic agent is introduced into the solvent with the polymer in the pre-formed mold. In a further specific example, during the electrospinning process, the therapeutic agent is electrosprayed onto the polymer being spun. In another example, a therapeutic agent is introduced into the myocardial patch before implantation. Therein, the therapeutic agent can be introduced into or onto the myocardial patch by immersing the patch in a solution containing the therapeutic agent, by applying a solution containing the therapeutic agent to the patch, or by vaporizing, sputtering, coating or similar methods. Secondary drugs can be, for example, anti-inflammatory drugs decreasing immune responses, drugs promoting cell growth, drugs promoting cell differentiation, neurotrophic drugs promoting neuronal growth and ameliorating arrhythmogenesis which may result from the infarction, chemo attractants promoting cellular migration or cellular infiltration into the patch, or any other drug helpful in the therapy or helpful to mitigate the side effects of the therapy.

The myocardial patch comprises at least one bordering lumen, wherein the bordering lumen is filled with adhesive to be discharged onto the epicardial surface. The adhesive is used to connect the myocardial patch with the heart in situ, such that the patch is held in place withstanding body fluids and the regular movement of the heart. An adhesive used for the myocardial patch must provide robust adhesion, be biocompatible and easily injectable. Preferably, the adhesive is biodegradable at least to some extent. Easy injection can be facilitated by suitable gelation kinetics and gelation times. For example, a gelation time in the range from about 30 seconds to about 120 seconds can be advantageous. Additionally or alternatively, the adhesive can be curable with a light source (e.g. ultra-violet light) located on the delivery device to achieve on-demand curing.

One embodiment comprises a myocardial patch, wherein the patch is adhered to the heart using an adhesive based on polyurethane. In one example, polyurethane engineered to have low hemolytic responses is used. In another example, a lysine-based sprayable urethane adhesive is used, for example the product TissuGlu^{®}. Polyurethane adhesives are typically used for fixation of vascular graft and bone, and in abdominoplasty surgery to avoid seroma formation.

One embodiment comprises a myocardial patch, wherein the patch is adhered to the heart using an adhesive based on PEG. In one example, tetra-succinimidyl PEG and tri-lysine amine is used, for example the product DuraSeal^{®}. In another example, acrylated PEG is used in connection with polyester primer and a photoinitiator, for example the product FocalSeal^{®}. In another example, glutaryl-succinimidyl ester and thiol terminated PEG, for example the product Coseal^{®}. PEG adhesives are typically used for preventing cerebrospinal fluid leakage after cranial operations and for reducing scar tissue and pain after microdiscectomy. Further, PEG adhesives are typically used to stop air leaks after lung surgeries and to seal suture lines and stop bleeding in vascular surgeries.

One embodiment comprises a myocardial patch, wherein the patch is adhered to the heart using an adhesive based on polyester. In one example, polyglycerol sebacate acrylate (also known as PGSA) is used. In another example, photocrosslinkable PGSA derivatives are used, for example the product SETALUM^{™} Sealant. Polyester adhesives are typically used for reducing the incidence of fluidic or air leaks and for repairing of vessels and heart defects.

One embodiment comprises a myocardial patch, wherein the patch is adhered to the heart using an adhesive based on polyvinyl alcohol. In one example, tyramine-modified PVA is used. Polyvinyl alcohol adhesives are typically used for hemorrhage control, wound closure and tissue anastomoses.

One embodiment comprises a myocardial patch, wherein the patch is adhered to the heart using an adhesive based on collagen. In one example, bovine collagen is used. Collagen adhesives are typically used for hemostasis.

One embodiment comprises a myocardial patch, wherein the patch is adhered to the heart using an adhesive based on gelatin. In one example, gelatin-resorcinol-formaldehyde-glutaraldehyde is used. In another example, gelatin and N-hydroxysuccinimide-ester functionalized poly-L-glutamic acid or disuccinimidyl tartrate is used. In another example, photocrosslinkable gelatin adhesive is used. In another example, gelatin and microbial transglutaminase is used. Gelatin adhesives are typically used for thoracic aortic dissections and hemostasis, seal surgical incisions in gastrointestinal tract surgeries, and repair retinal tissues.

One embodiment comprises a myocardial patch, wherein in patch is adhered to the heart using an adhesive based on albumin. In one example, bovine albumin and glutaraldehyde is used, for example the product BioGlue^{®}. In another example, human albumin and a NHS-activated PEG is used, for example the product Progel^{®}.

One embodiment comprises a myocardial patch, wherein the patch is adhered to the heart using an adhesive based on chitosan. In one example, lactobionic acid and azide functionalized chitosan is used. In another example, tyrosine-modified chitosan, HPR and hydrogen peroxide is used. In another example, thiol-containing chitosan and maleimide containing epsilon-polylysine is used. Chitosan adhesives are typically used for wound closure and hemostasis.

One embodiment comprises a myocardial patch, wherein the patch is adhered to the heart using an adhesive based on dextran. In one example, aldehyde-containing dextran and amine-containing PEG or polylysine crosslinkers are used. Dextran adhesives are typically used to stop air leaks after lung surgeries.

One embodiment comprises a myocardial patch, wherein the patch is adhered to the heart using an adhesive based on chondroitin sulfate. In one example, aldehyde-bearing chondroitin sulfate and polyvinyl alcohol-co-vinyl amine is used. In another example, methacrylate and aldehyde functionalized chondroitin sulfate. NHS-activated chondroitin sulfate and amine-containing PEG. Chondroitin sulfate adhesives are typically used for sealing corneal incisions, binding to native cartilage tissue, and wound closure.

The reservoir lumen is typically filled with a carrier material containing a therapeutic agent. Therein, the carrier material is typically a saline, a hydrogel, or an adhesive. A desirable contribution to the overall stability of the myocardial patch can be facilitated by a suitable carrier material. Furthermore, a carrier material can be selected or engineered to comprise an advantageous gelation mechanism, for example for improved controllability or improved gelation rate.

The function of the carrier material is the temporary retention of a therapeutic agent to allow for the bulk of the therapeutic agent to be released only once the final, implanted position on the epicardial surface of the heart is secured and to enable the controlled release of the therapeutic agent to the adjacent region to where the patch is adhered to the epicardial surface of the heart. Migration of the therapeutic agent from the carrier material to the targeted, adjacent epicardial surface of the heart occur in a controlled manner by one or more of the following mechanisms:
(1) diffusion of the therapeutic agent from the carrier material in the reservoir lumen through an initially permeable configured first layer,
(2) diffusion of the therapeutic agent, which was set free during carrier material degradation caused by naturally occurring digestive agents from the host immune system. The digestive agents are able to cross the first layer due to its initially permeable configuration,
(3) active migration of the therapeutic agent, for example stem cells, from the carrier material through the initially permeable configured first layer, and
(4) biodegradation of the material of the initially less permeable configured first layer, causing increased permeability in the first layer allowing for easier passage of the therapeutic agent contained in the carrier material through diffusion or active migration.

Some embodiments comprise a myocardial patch, wherein the reservoir lumen is filled with a carrier material based on one or more natural substances.

One embodiment comprises a myocardial patch, wherein the reservoir lumen is filled with a carrier material based on collagen. Therein, the gelation of the carrier material is induced and/or influenced thermally. Gelation is typically slow, however. By using a carrier material based on collagen, the carrier material can be biocompatible and biodegradable.

One embodiment comprises a myocardial patch, wherein the reservoir lumen is filled with a carrier material based on fibrin. Therein, the gelation of the carrier material is induced and/or influenced by peptide self-assembly. Gelation is typically slow and the carrier material provides little mechanical strength. By using a carrier material based on fibrin, the carrier material can be biocompatible. Further, the carrier material is biodegradable, wherein the degradation rate in vivo can be too fast in some cases. Further, the carrier material is highly available. Further, vascularization can be better facilitated by a carrier material based on fibrin.

One embodiment comprises a myocardial patch, wherein the reservoir lumen is filled with a carrier material based on Matrigel^{™}. Therein, the gelation of the carrier material is induced and/or influenced thermally. A carrier material based on Matrigel^{™} is not suitable in cases when it might me tumorigenic. By using a carrier material based on Matrigel^{™}, the carrier material can be biocompatible. Further the carrier material resembles to native ECM and provides fast vascularization.

One embodiment comprises a myocardial patch, wherein the reservoir lumen is filled with a carrier material based on hyaluronic acid. Therein, the gelation of the carrier material is induced and/or influenced by a redox inhibitor. Gelation via crosslinks requires modifications in the material as typically available, however. By using a carrier material based on hyaluronic acid, the carrier material can be biocompatible and biodegradable.

One embodiment comprises a myocardial patch, wherein the reservoir lumen is filled with a carrier material based on collagen. Therein, the gelation of the carrier material is induced and/or influenced thermally. Gelation is typically slow, however. By using a carrier material based on collagen, the carrier material can be biocompatible and biodegradable.

One embodiment comprises a myocardial patch, wherein the reservoir lumen is filled with a carrier material based on chitosan. Therein, the gelation of the carrier material is induced and/or influenced thermally and dependent on presence of glycerol phosphate. By using a carrier material based on chitosan, the carrier material can be biocompatible and biodegradable.

One embodiment comprises a myocardial patch, wherein the reservoir lumen is filled with a carrier material based on alginate. Therein, the gelation of the carrier material is induced and/or influenced depending on presence of Ca2+. By using a carrier material based on collagen, the carrier material can comprise mechanical strength, biocompatibility and biodegradability.

Some embodiments comprise a myocardial patch, wherein the reservoir lumen is filled with a carrier material based on one or more synthetic substances.

One embodiment comprises a myocardial patch, wherein the reservoir lumen is filled with a carrier material based on PEG. Non-limiting examples comprise carrier materials based on mPEG-PCL-mPEG with alpha-cyclodextrin, 8a-PEG-VS with a cysteine-flanked peptide, Oligo (PEG fumarate) with PEGDA, or PVL-PEG-PVL. Therein, the gelation of the carrier material is induced and/or influenced by peptide self-assembly. A carrier material based on PEG is not injected into the myocardial patch after implantation, but needs to be placed in the patch in advance. Further, the carrier material is typically bio-inert, i.e. it does not tend to interact chemically or biologically with the surrounding tissue. Thereby, the carrier material comprises low cell adhesion and is biocompatible.

One embodiment comprises a myocardial patch, wherein the reservoir lumen is filled with a carrier material based on PNIPAAm. Non-limiting examples comprise carrier materials based on PNIPAAm-co-(HEMA-PCL-grafted dextran), PNIPAAm-co-AK-co-HEMApTMC, or PNIPAAm-co-AAc with a peptide cross-linker and RGD-grafted pAAc. Therein, the gelation of the carrier material is induced and/or influenced thermally. By using a carrier material based on PNIPAAm, the carrier material can be biodegradable, though slowly. Further, PNIPAAm comprises extensibility of its network chains.

Further embodiments comprise a myocardial patch, wherein the reservoir lumen is filled with a carrier material based on or comprising one or more of chitin, polyvinylpyrrolidone, polylac ticpolyglycolic acid, polyanhydride, polyurethane, dimethylpolysiloxane, ethylene vinyl acetate, polymethyl methacrylate, polyamide, polycarbonate, polyester, polyethylene, polypropylene, polyhdroxybutyrate, polystyrene, polyvinyl chloride, polytetrafluoroethylene, and cellulose acetate.

Alternatively, one embodiment comprises a myocardial patch, wherein the reservoir lumen is filled with a non-gel carrier material. In one example, the reservoir lumen is filled with a tissue engineering construct like a 3D polyurethane scaffold, like for example the product Biomerix^{™}.

One embodiment comprises a myocardial patch and an injection system, wherein the injection system is in fluid communication with at least one of a bordering lumen and a reservoir lumen. The injection system is preferably charged with a carrier material to be filled into the reservoir lumen and/or one or more adhesive components to be filled into at least one bordering lumen. Alternatively or additionally, the injection system is connected to a reservoir of a carrier material to be filled into the reservoir lumen and/or one or more adhesive components to be filled into at least one bordering lumen. The injection system can comprise an infusion pump or an auto-injector for a controlled injection rate of a carrier material into the reservoir lumen and/or one or more adhesive components into the bordering lumen. Therein, a controlled flow of carrier material or adhesive can be ensured. Further, a predetermined pre-gelling timing of a carrier material or a pre-determined mixing ratio of components of adhesive can be maintained for optimized filling of the reservoir lumen and/or the bordering lumen and to avoid undesired clogging. By using an injection pump or an auto-injector, a defined and reliable injection process can be ensured, especially in case of materials which are more difficult to manage by manual injection.

Implanted onto a patient heart surface, a myocardial patch can have a number of beneficial effects on to patient heart. For example, fibrosis induced by the foreign material can strengthen the epicardium. Parts of the patch can be made of materials promoting cell growth, cell differentiation, or neuronal growth through their presence. Degradable scaffold structures provide mechanical support by promoting ingrowth, wherein stretched cardio-myocytes that cannot contract enhance the effect. Additionally or alternatively, the myocardial patch releases a therapeutic agent at a predefined rate to the heart, in particular to the epicardium and/or to the pericardium. Therein, one or more of a variety of therapeutic agents are contained in a carrier material or in a therapeutic product, depending on the therapy target.

One embodiment comprises a myocardial patch, wherein the myocardial patch is configured to contain and release a therapeutic agent based on stem cells. Preferably, the therapeutic agent comprises cardiac or cardiopoietic stem cells, as extensive preclinical and clinical trials have identified. Alternatively or additionally, further stem cells, for example myoblasts, mesenchymal stem cells, embryonic stem cells, can be useful for cardiac regeneration. Stem cells can be delivered to the patient heart by being loaded into a suitable hydrogel carrier material fixed to the epicardium, as described herein. Alternatively, stem cells can be delivered to the patient heart by being seeded into a porous scaffold fixed to the epicardium, as described herein. By using a myocardial patch configured to contain and release a therapeutic agent based on stem cells, very good cardiac regeneration can be achieved.

One embodiment comprises a myocardial patch, wherein the myocardial patch is configured to contain and release a therapeutic agent based on Prostaglandin E2 (also known as PGE2). PGE2 is rapidly metabolized in-vivo and therefore needs to be replenished repeatedly. By using a therapeutic agent based on PGE2, cardiomyocyte replenishment at an infarct border zone is enhanced according to a murine model of myocardial infarction. Therefore, a myocardial patch configured to contain and release a therapeutic agent based on PGE2 is useful for cardiac regeneration after myocardial infarction.

One embodiment comprises a myocardial patch, wherein the myocardial patch is configured to contain and release a therapeutic agent based on prostaglandin I2. Prostaglandin I2 is rapidly metabolized in-vivo and therefore needs to be replenished repeatedly. Since prostaglandin I2 can cause significant side-effects when metabolized systemically, prostaglandin I2 is preferably delivered locally to the therapy site only. By using a therapeutic agent based on Prostaglandin I2, myocardial expression of cardioprotective HGF, VEGF, SDF-1 and G-CSF is facilitated. Therefore, a myocardial patch configured to contain and release a therapeutic agent based on prostaglandin I2 is useful for treatment of hypertension.

One embodiment comprises a myocardial patch, wherein the myocardial patch is configured to contain and release a therapeutic agent based on pyrvinium pamoate (also known as PP).

PP can be cytotoxic to cardiomyocytes in an infarct border zone when perfusion is limited or CSCs are naturally present in a hypoxic niche. Therefore, timing of the application of PP is critical. PP is an antihelmintic, which inhibits HADH-fumarate reductase activity essential for the anaerobic respiration of parasitic worms. By using a myocardial patch configured to contain and release a therapeutic agent based on PP, anti-fibrotic therapy within 24 hours after a myocardial infarction is enabled.

One embodiment comprises a myocardial patch, wherein the myocardial patch is configured to contain and release a therapeutic agent based on dipeptidylpeptidase IV (also known as DPP-IV) inhibition. DPP-IV inhibitors are rapidly metabolized in-vivo and therefore need to be replenished repeatedly. By using a therapeutic agent based on DPP-IV inhibition, recruitment of CXCR4+ circulating stem cells is facilitated. Therefore, a myocardial patch configured to contain and release a therapeutic agent based on DPP-IV inhibition is useful for cardiac regeneration.

One embodiment comprises a myocardial patch, wherein the myocardial patch is configured to contain and release a therapeutic agent based on modified messenger RNA (also known as modmRNA), for example the product Lipofectamine^{™}. For modmRNA therapy, timing and duration of release of the therapeutic agent is critical. By using modmRNA, cardiomyocytes could be transfected with high efficiency, producing advantageous effects to the heart. Further, by using modmRNA, a risk of insertional mutagenesis, as present with conventional DNA therapy, is avoided. Therefore, a myocardial patch configured to contain and release a therapeutic agent based on modmRNA is promising to become an effective therapy approach for cardiac regeneration.

One embodiment comprises a myocardial patch, wherein the myocardial patch is configured to contain and release a therapeutic agent based on MicroRNA targeting (also known as miRs). miRs pose unpredicatable off-target effects and can therefore not be administered systemically. Therefore, miRs are preferably delivered locally to the therapy site only. Since miRs are extensively involved in the cardiac development and postnatal desease processes including ventricular remodeling and fibrosis following infarction and processes with therapeutic applicability in acute infarction such as angiogenesis or myocardial regeneration, a myocardial patch configured to contain and release a therapeutic agent based on miRs could be very effective and safe for cardiac regeneration.

Some embodiments comprise a myocardial patch, wherein the myocardial patch is configured to contain and release a therapeutic agent based on direct reprogramming of cardiac fibroblasts to functional cardiomyocytes or cardiac progenitor cells. The heart contains a large pool of fibroblasts necessary for the normal function of the heart. Therefore, if affection of such necessary fibroblasts is avoided, a myocardial patch configured to contain and release a therapeutic agent based on direct reprogramming of cardiac fibroblasts could be effective and safe for cardiac regeneration.

Some embodiments comprise a myocardial patch, wherein the myocardial patch is configured to contain and release a therapeutic agent based on growth factors and/or proteins. Therein, modified peptides and proteins can enable cardiac repair through activation of endogenous cardiac progenitor cells present at an injury site. Further, modified peptides and proteins can enable the induction of cardiomyocyte proliferation and the recruitment of progenitor cells to damaged myocardium or of functional cells able to trigger neovascularisation. Therefore, a myocardial patch configured to contain and release a therapeutic agent based on growth factors and/or proteins could be useful for cardiac regeneration.

A myocardial patch configured to contain and release a therapeutic agent based on one of the drugs described herein or not described herein is easier from a regulatory side and for upscaling of therapy success. However, some embodiments comprise a myocardial patch, wherein the myocardial patch is configured to contain and release a therapeutic agent based on a number of drugs described herein or not described herein. Therefore, more control over the therapy targets can be exercised and multiple problems associated with one or more diseases can be addressed at once.

One embodiment comprises a myocardial patch, wherein the myocardial patch is configured to contain and release at least one anti-inflammatory drug. In one example, the anti-inflammatory drug is a nonsteroidal anti-inflammatory agent, for example one of salicylic acid derivatives (aspirin), para-aminophenol derivatives(acetaminophen), indole and in-dene acetic acids (indomethacin), heteroaryl acetic acids (ketorolac), arylpropionic acids (ibuprofen), anthranilic acids (mefenamic acid), enolic acids (oxicams) and alkanones (nabumetone). In another example, the anti-inflammatory drug is a steroidal anti-inflammatory agent, for example corticosteriods and biologically active synthetic analogs with respect to their relative glucocorticoid (metabolic) and mineralocorticoid (electrolyte-regulating) activities. In yet another example, the anti-inflammatory drug is one of the group of autocoid antagonists such as all histamine and bradykinin receptor antagonists, leukotriene and prostaglandin receptor antagonists, platelet activating factor receptor antagonists, or any other suitable anti-inflammatory drug.

One embodiment comprises a myocardial patch, wherein the myocardial patch is configured to contain and release at least one antimicrobial drug. In one example, the antimicrobial drug is one of the group of antibiotics (e.g. antibacterial), antiviral agents, antifungal agents, and anti-protozoan agents. In particuar, the antimicrobial drug can be one of the group of sulfonamides, trimethoprim-sulfamethoxazole, quinolones, penicillins, and cephalosporins. One embodiment comprises a myocardial patch, wherein the myocardial patch is configured to contain and release at least one antineoplastic drug. In one example, the antineoplastic drug is one of the group of antibiotics (e.g. antibacterial), antiviral agents, antifungal agents, and anti-protozoan agents. In particuar, the antimicrobial drug can be one of the group of sulfonamides, trimethoprim-sulfamethoxazole, quinolones, penicillins, and cephalosporins.

One embodiment comprises a myocardial patch, wherein the second layer is an elastomer made by using solvent casting and salt leaching. Hereto, a polymer is dissolved into an organic solvent. Subsequently, the solution is casted into a pre-formed mold containing porogens, i.e. small particles of a suitable material with a defined size. Porogens, which can be for example saccharose, inorganic salts, gelatin spheres, paraffin spheres, are responsible for a porosity of the final elastomer. After casting the solution into the mold containing porogens, the solvent is evaporated, leaving a polymer composition wherein the porogens are randomly distributed. The polymer composition is immersed into a solvent configured to dissolve the porogens, but not the polymer. Thus, a porous, sheet-like material is yielded.

One embodiment comprises a myocardial patch, wherein the second layer is an elastomer made by using thermally induced phase separation (TIPS). Hereto, a polymer is dissolved into an organic solvent. Subsequently, the solution is injected into a pre-formed mold. The mold is cooled to a low temperature, for example below -80 degrees Celsius. Finally, the solvent is extracted from the mold by ethanol. In one example, the polymer PEU (10% w/v) is dissolved in the solvent DMSO at 80 degrees Celsius. Then, the solvent is injected into a pre-formed mold and cooled down to -80 degrees Celsius over three hours. Thereafter, the mold is immersed in ethanol at 4 degrees Celsius for seven days. Finally, the mold is freeze-dried for 48 hours to yield a porous material.

One embodiment comprises a myocardial patch, wherein the first layer is an elastomer made by using electrospinning. Hereto, a polymer is dissolved into a solvent in a reservoir. Alternatively, a polymer can be melted, a polymer can be suspended in a suspension, or a polymer can be contained in any other form in a fluid. The reservoir comprises a metering pump. Further, the reservoir comprises a needle, a pipette tip or a similar small orifice, which is in electrical contact with an electrode. A second electrode is in electrical contact with a rotating mandrel, whereby the electrodes are connected to a high voltage source. Alternative to the rotating mandrel, a collector screen or another suitable target can be used. When the polymer-containing fluid is pushed through the small orifice by the metering pump, a steady flow of fluid is generated resembling that of a fountain. By applying a high voltage, preferably in the range from about 2 kV to about 16 kV, to the electrode in electrical contact with the orifice, and biasing the rotating mandrel with a voltage in the range from about -2 kV to about -10 kV, a jet of fluid is ejected at the orifice towards the rotating mandrel. During its flight to the mandrel, the fluid is preferably turned into a polymer fiber. For example, in the case that the fluid is a polymer solution, the solvent evaporates on the way. In another example, in the case that the fluid is a polymer melt, the melt cools down on the way. Eventually, a non-woven, porous mesh of polymer fiber accumulates on the rotating mandrel. The properties of the electrospun elastomer can be varied by varying the geometry of the electrospinning apparatus, e.g. the distance of the target from the ostrice, the selection of the target, the selection of the ostrice, the humidity and the voltages applied. For example, a higher humidity typically leads to a higher porousity of the electrospun elastomer. Further, the fluid jet can be focused using a focusing ring biased with a voltage in the range from about 1 kV to about 10 kV arranged between the orifice and the target.

The myocardial patch comprises a reservoir lumen defined by the first layer and the second layer. Therein, the reservoir lumen is bound by a joint between the first layer and the second layer. Typically, outside of the reservoir lumen and the bordering lumen, the first layer and the second layer are permanently connected using at least one of a variety of bonding techniques.

One embodiment comprises a myocardial patch comprising a first layer and a second layer, wherein the first layer and the second layer are connected using a thermal bonding method. Preferably, the first layer and the second layer are 3D laser welded or using an appropriately heated and geometrically formed stamp.

Another embodiment comprises a myocardial patch comprising a first and a second layer, wherein the first layer and the second layer are connected using a chemical bonding method.

Preferably, the myocardial patch 3 is applied to the heart 1 in a minimally-invasive operation via a small opening in the pericardial sac 4. Hereto, the patch 3 is collapsed for delivery to the heart 1 and expanded remotely when in position.

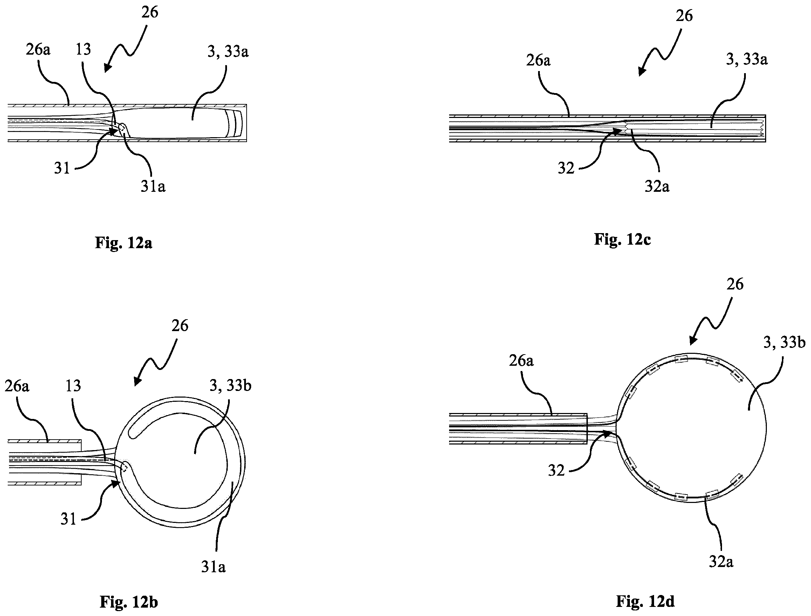

**Fig. 12a** shows an embodiment comprising a tube 26a of a delivery device 26 containing a collapsed support structure 26b and a collapsed myocardial patch 33a. Thereby, the support structure 31 comprises an inflatable member 31a, which is releasably connected to the patch 3 along its circumference. The support structure 31 is configured to expand the myocardial patch 3 from its collapsed state into an expanded state.

**Fig. 12b** shows an embodiment comprising a tube 26a of a delivery device 26, whereby the support structure 31 releasably connected to an expanded myocardial patch 33b along its circumference as a whole has been advanced out of the tube 26a of the delivery device 26. The support structure 31 comprising an inflatable member 31a is connected to supply tubes 13 such as a gas supply tube, wherein the supply tubes lead into the tube of the delivery device 26. By inflating gas into the inflatable member 31a, the entire patch 3 is expanded.

**Fig. 12c** shows an embodiment comprising a tube 26a of a delivery device 26 containing a collapsed support structure 32 and a collapsed myocardial patch 33a. Thereby, the support structure 32 comprises a shape memory structure 32a, which is releasably connected to the patch 3 along its circumference. The support structure 32 is configured to expand the myocardial patch 3 from its collapsed state into an expanded state.

**Fig. 12d** shows an embodiment comprising a tube 26a of a delivery device 26, whereby the support structure 32 releasably connected to the expanded myocardial patch 33b along its circumference as a whole has been advanced out of the tube 26a of the delivery device 26.

The support structure 32 comprises a shape memory structure 32a along the circumference of the patch 3. A shape memory structure 32a could for example be a Nitinol structure having a collapsed state below a transformation temperature and an expanded state above the temperature. The patch 3 is then cooled significantly below the switching temperature before delivery to the therapy site. When the patch 3 is pushed out of the delivery device 26, it warms up to a temperature above the transformation temperature of the shape memory structure 32a, thereby expanding. With the expansion of the shape memory structure 32a the entire patch 3 is expanded.

One embodiment comprises a myocardial patch 3 and a support structure releasably attached to the patch, wherein the support structure comprises a hollow member which is configured to receive a catheter wire or stylet which expands the myocardial patch 3 from its collapsed state into an expanded state. In one embodiment, one or more bordering lumens can serve as hollow member. In other embodiments, the hollow member is a structure different from the one or more bordering lumens.

One embodiment comprises a myocardial patch 3 and a support structure releasably attached to the patch, wherein the support structure comprises a flexible foam material which is configured to expand the myocardial patch 3 from its collapsed state into an expanded state once restrictions imposed by the delivery device are removed. Once the foam material is released from the delivery device, it expands by itself due to the residual stressed in the foam material.

One embodiment comprises a myocardial patch 3, wherein the myocardial patch 3 comprises a flexible foam material which is configured to expand the myocardial patch 3 from its collapsed state into an expanded state. The foam material is kept collapsed by the restrictions imposed by the delivery device. Once the foam material is released from the delivery device into the pericardial sac, it expands by itself due to the residual stressed in the foam material. Preferably, the foam material makes up the second layer 18.

Once the myocardial patch 3 is delivered to the therapy site, the patch is expanded, optional protective layers as described before are removed, and the patch is placed on the epicardial surface 2. Subsequently, the patch is adhered to the epicardial surface 2. Once the myocardial patch 3 adheres sufficiently to the heart surface, the delivery device can be released. To this end, the delivery device can be connected to the patch using one of a variety of releasable connections.

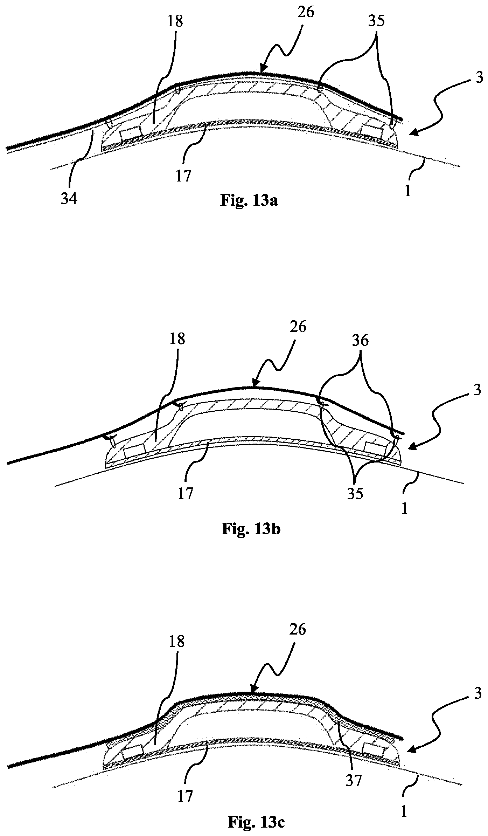

**Fig. 13a** shows an embodiment comprising a myocardial patch 3 and a delivery device 26, wherein the myocardial patch 3 is held to the delivery device 26 by a string 34 threaded through corresponding eyelets 35. When the patch 3 adheres to the heart 1, the string 34 is pulled to release the patch 3 from the delivery device 26. Subsequently, the delivery device 26 can be removed without causing significant mechanical disturbance to the adhered patch 3. By using a connection comprising a string 34, an easy and fairly fail-proof method to connect and release the myocardial patch 3 to and from the delivery device 26 is provided.

**Fig. 13b** shows an embodiment comprising a myocardial patch 3 and a delivery device 26, wherein the myocardial patch 3 is held to the delivery device 26 by hooks 36. Preferably, the hooks 36 are part of the delivery device 26 and corresponding eyelets 35 are provided on the myocardial patch 3. Alternatively, the hooks 36 can be part of the myocardial patch 3 and corresponding eyelets 35 are provided on the delivery device 26. Eyelets 35 can also be omitted if provision is made in the second layer 18 for lodging of the hooks 36, corresponding to the position of the hooks 36 on the delivery device. Corresponding to the hooks 36, eyelets 35 are preferentially provided. However, the hooks 36 fixate the patch 3 when moved in direction of the hooks 36 or any direction orthogonal to it. When moved against the direction of the hooks 36, the hooks 36 disengage from the myocardial patch 3, thereby releasing the patch 3 from the delivery device 26 without causing significant mechanical disturbance to the adhered patch 3. By varying the design of the hooks 36, a trade-off between a secure connection and easy release can be made. Using a connection comprising hooks 36, an easy method to connect and release the myocardial patch 3 to and from the delivery device 26 is provided, which necessitates minimal extra structures.

**Fig. 13c** shows an embodiment comprising a myocardial patch 3 and a delivery device 26, wherein the myocardial patch 3 is held to the delivery device 26 by a weak adhesive 37. The adhesive fixates the patch 3 when moved careful. By a special maneuver, for example by introducing a torsion force on the delivery device 26 against the myocardial patch 3, the adhesion can be severed and eventually broken, thereby releasing the patch 3 from the delivery device without causing significant mechanical disturbance to the adhered patch 3. By varying the strength of the adhesive and the shape of the adhesion area, a secure connection and easy release can be pursued. Using a connection comprising adhesive, an easy method to connect and release the myocardial patch 3 to and from the delivery device 26 is provided, which necessitates minimal extra structures.

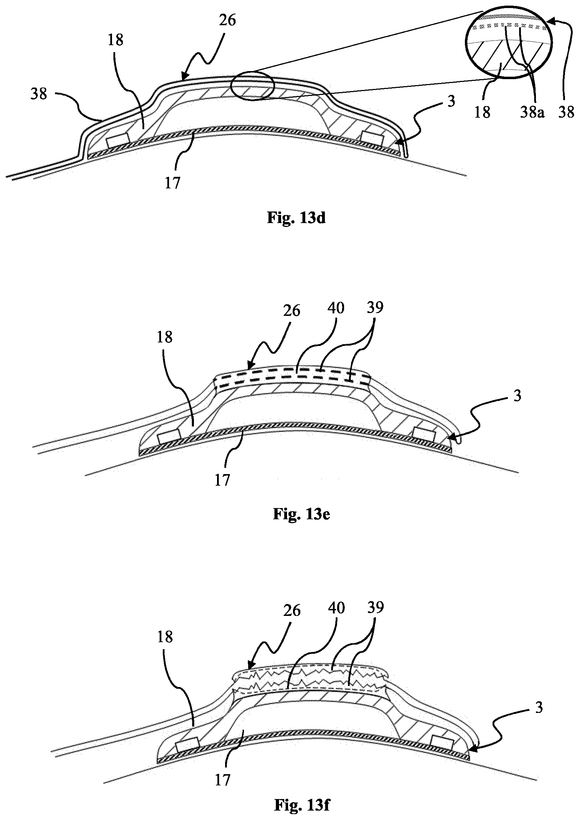

**Fig. 13d** shows an embodiment comprising a myocardial patch 3 and a delivery device 26, wherein the myocardial patch 3 is held to the delivery device 26 by suction based on a vacuum provided to the delivery device 26. An enclosing element 38 is put over at least a part of the second layer 18, such that a tight space is defined between the enclosing element 38 and the second layer 18. The enclosing element 38 comprises openings 38a on a side facing the second member 18 such that vacuum can be drawn within the hollow enclosing element 38, thereby sucking out the air from the tight space between the second layer 18 and the enclosing element 38. The vacuum in this space fixates the patch 3 as long as the vacuum is maintained. By releasing the vacuum, preferably on the operator side of the delivery device 26, the suction is interrupted, thereby releasing the patch 3 from the delivery device 26 without causing significant mechanical disturbance to the adhered patch 3. Using a connection comprising a vacuum, a secure and flexible method to connect and release the myocardial patch 3 to and from the delivery device 26 is provided.

**Fig. 13e** shows an embodiment comprising a myocardial patch 3 and a delivery device 26 comprising one or more distal balloons 40, wherein the myocardial patch 3 is connected to the delivery device 26 by tearable fleeces 39. By inflating the one or more balloon 40, the one or more balloon 40 causes the fleeces 39 to tear, thereby separating the delivery device 26 from the myocardial patch 3.

**Fig. 13f** shows an embodiment comprising a myocardial patch 3 and a delivery device 26 comprising one or more distal balloons 40, wherein the distal balloons 40 are inflated, thereby having teared the fleece 39 connecting the myocardial patch 3 to the delivery device 26. Thus, the patch 3 is released from the delivery device 26 without causing significant mechanical disturbance to the adhered patch. Using a connection comprising tearable fleeces 39, a secure and flexible method to connect and release the myocardial patch 3 to and from the delivery device is provided.

Preferably, the myocardial patch 3 comprises supply lines for in-situ supply of adhesive to the bordering lumen 7 and in-situ supply of a carrier material 28 with therapeutic agent to the reservoir lumen 8 after implantation of the patch to the heart. The supply lines are necessary only for an initial filling of the bordering lumen 7 and of the reservoir lumen 8 and are obsolete from there on. Thus, the supply lines are preferably attached to the respective ports of the myocardial patch 3 using one of a variety of releasable connections.

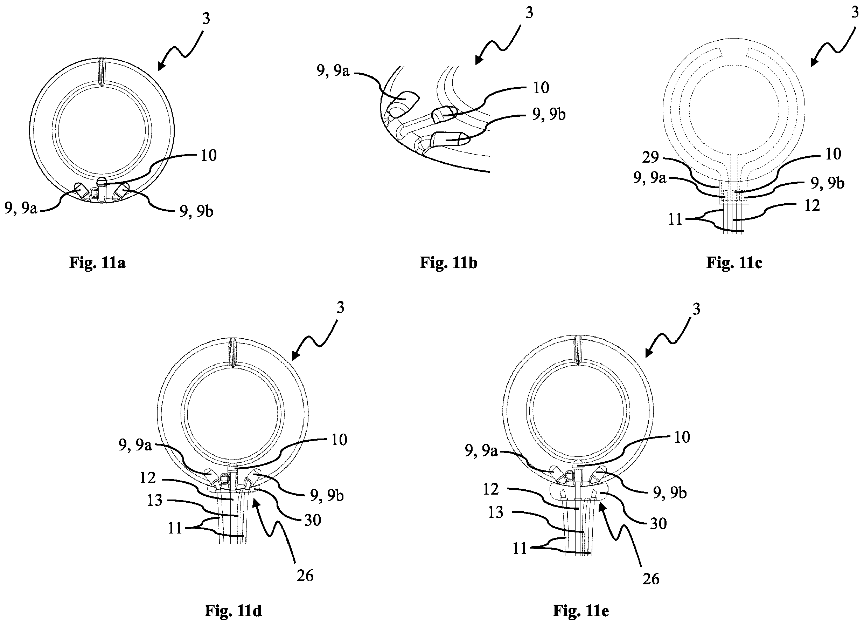

**Fig. 11a** and **Fig. 11b** show an embodiment comprising a myocardial patch 3, wherein the myocardial patch 3 comprises a reservoir lumen port 10, a first bordering lumen port 9a and a second bordering lumen port 9b. Further embodiments can comprise any number of ports (9, 10) in accordance with the different embodiments described herein. Each port (9, 10) is made of a flexible material, for example a flexible polymer material. A supply line, which is not depicted, is introduced into each of the ports (9, 10) and held in place by the friction resulting from a tight fit of the supply line in the port (9, 10). Therefore, the port (9, 10) is in fluid communication with the supply line and no fluid can leak from the connection between the port (9, 10) and the supply line. The connection can withstand against tension of the supply line up to a certain tension threshold. The connection can be released by applying a tension to the supply line beyond the tension threshold while stabilizing the myocardial patch 3 using at least a part of the delivery device or using a sheath over the supply line. Alternatively, the connection can be released by applying torsion to the supply line and applying tension to the supply line at the same time, such that the sum of both overcomes the friction forces of the connection. In other words, the supply line is twisted out of the port (9, 10). Either way, the force which can reasonably be applied to the supply line is limited. Therefore, the connection cannot be configured for too high of a tension threshold.

In further embodiments, the ports (9, 10) may be arranged adjacent each other, with an inclination towards the middle port. When supply lines are connected to the ports, the supply lines join in the middle and can easily be interconnected as described before. Alternatively, the ports (9, 10) can be arranged adjacent each other, in parallel. This avoids that supply lines come into the way of each other. Alternatively, the ports (9, 10) can be arranged in a distance from one another, for example at different ends of the myocardial patch 3. This can be necessary to achieve a specific layout of the myocardial space, or to save areal space on the myocardial patch 3.

**Fig. 1** shows one embodiment comprising a myocardial patch 3 and one or more supply lines (11, 12), wherein the supply lines (11, 12) corresponding to the various ports are interconnected, for example glued to each other, proximal of the respective ports. Alternatively, the supply lines (11, 12) can be inter-connected by being produced inter-connected, by being clammed, by being fixed using a thread, by being enclosed using a common sheath 16, preferably a flexibly sheath or a shrinking sheath, or other common methods to join multiple tubes. Preferably, the point where the supply lines begin to be inter-connected is between 5 millimeters and 5 centimeters proximal of the respective ports. When tension to one or more of the supply lines (11, 12) is applied proximal the inter-connection beginning point, the supply line (11, 12) is not released from the port accidentally. Only if the tensile load exceeds the sum of the tension thresholds of all supply lines (11, 12), supply lines (11, 12) are pulled out of the respective ports. Hence, by inter-connecting the supply lines, accidental release of the supply lines from their respective ports is effectively avoided. Still, the supply lines (11, 12) can be released fairly easily by applying tension or a combination of tension and torsion distally of the inter-connection beginning point, for example using a delivery device.

One preferably embodiment comprises a myocardial patch 3 and one or more supply lines (11, 12), wherein the supply lines (11, 12) corresponding to the various ports (9, 10) are inter-connected, for example glued to each other, proximal of the respective ports (9, 10). Alternatively, the supply lines (11, 12) can be inter-connected by being produced interconnected, by being clammed, by being fixed using a thread, by being enclosed using a common sheath 16, preferably a flexibly sheath or a shrinking sheath, or other common methods to join multiple tubes. Preferably, the point where the supply lines (11, 12) end to be inter-connected is outside of the patient body. The supply lines (11, 12) are inter-connected along their length from the beginning point to the end point. Alternatively, the supply lines (11, 12) can be interconnected only at the beginning point and at the end point, or at the beginning point, the end point and intermediate points between the beginning point and the end point, or along their length in one or more intervals between the beginning point and the end point, comprising the beginning point and the end point. By inter-connecting the supply lines (11, 12) outside of the patient body, the supply lines (11, 12) can be easily controlled to not interfere with the operand during the transplantation, and the inter-connection can easily be made strong enough for any occurring forces, e.g. tensions or torsions.

**Fig. 11c** shows an embodiment comprising a myocardial patch 3, wherein the myocardial patch 3 comprises a reservoir lumen port 10, a first bordering lumen port 9a and a second bordering lumen port 9a. Further embodiments can comprise any number of ports (9, 10) in accordance with the different embodiments described herein. The ports (9, 10) are housed in a housing part 29 of the myocardial patch 3 protruding from the rest of the patch 3. A supply line (11, 12) is introduced into each of the ports (9, 10) and held in place by the friction resulting from a tight fit of the supply line in the port (9, 10). Therefore, the port (9, 10) is in fluid communication with the supply line (11, 12) and no fluid can leak from the connection between the port (9, 10) and the supply line (11, 12). The connection between the ports (9, 10) and the supply lines (11, 12) is configured to withstand high tensile loads and is not intended to be released. Instead, the supply lines (11, 12) are separated from the myocardial patch 3 by separating the housing part 29 including the ports (9, 10) from the patch 3. Therein, the housing part 29 can be cut off of the patch 3 using part of the delivery device used to deliver the patch 3 to the therapy site. Alternatively, the housing part 29 can be cut off of the patch 3 using a separate device introduced to the therapy site. Alternatively, the housing part 29 can be twisted off the patch 3 by applying torsion to the housing 29, wherein an abutment stabilizes the myocardial patch 3 adjacent the housing 29 against the torsion. Alternatively, the housing 29 can be teared off the patch 3 by applying tension to the housing 29, wherein the myocardial patch 3 adjacent the housing 29 is stabilized against the tension. By separating the supply lines (11, 12) from the myocardial patch 3 by separating a housing 29 including the ports (9, 10) from the myocardial patch 3, a very stable connection between the supply lines (11, 12) and the ports (9, 10) is allowed. By separating the housing part 29 including the ports (9, 10) from the myocardial patch 3 by cutting the housing part 29 off, accidentally separating the supply lines (11, 12) from the patch 3 is avoided. The housing part 29 protruding from the rest of the patch 3 is advantageous to facilitate cutting off the housing part 29 and to avoid severing the patch 3 in the process.

**Fig. 11d** and **Fig. 11e** show an embodiment comprising a myocardial patch 3, wherein the myocardial patch 3 comprises a reservoir lumen port 10, a first bordering lumen port 9a, a second bordering lumen port 9b, and a delivery device 26 comprising a port balloon 30. Further embodiments can comprise any number of ports (9, 10) in accordance with the different embodiments described herein. The ports (9, 10) are arranged in vicinity to each other and with a common orientation. A supply line (11, 12) is introduced into each of the ports (9, 10) and held in place by the friction resulting from a tight fit of the supply line (11, 12) in the port (9, 10). Therefore, the bordering lumen port (9a, 9b) is in fluid communication with the bordering lumen supply line 11 and the reservoir lumen port 10 is in fluid communication with the reservoir lumen supply line 12, such that no fluid can leak from the connection between the port (9, 10) and the supply line (11, 12). The connection can withstand tension of the supply line (11, 12) up to a certain tension threshold. The supply lines (11, 12) are connected to a first end of the port balloon 30 close to the ports (9, 10), whereby all supply lines (11, 12) are inter-connected. A second end of the port balloon 30 is leaned against the myocardial patch 3, preferably distal of the ports (9, 10). When the balloon 30 is inflated (see Fig. 11e), for example using the delivery device 26 or using a balloon supply line 13, the first end of the balloon 30 is pushed away from the second end of the balloon 30 and thereby the supply lines (11, 12) are pushed away from the myocardial patch 3 in a proximal direction, releasing the connections of the supply lines (11, 12) to the respective ports (9, 10). By separating the supply lines (11, 12) from the myocardial patch 3 using a port balloon 30, a stable connection between the supply lines (11, 12) and the ports (9, 10) is allowed, while releasing the connection is easy and fail-proof.

One embodiment comprises a myocardial patch 3 and a delivery device, wherein the delivery device comprises a two-stage circular balloon. Therein, the myocardial patch 3 is collapsible and configured to be expanded by inflating the two-stage circular balloon to a first state. Further, the myocardial patch 3 is connected to the delivery device by tearable fleeces 39. By inflating the two-stage circular balloon further to a second state, the balloon separates the delivery device from the myocardial patch 3, causing the fleeces to tear. Alternatively or additionally, the two-stage circular balloon is connected to the supply lines. By inflating the two-stage circular balloon to a second stage, the balloon separates the supply lines from the respective ports (9, 10), thereby releasing the myocardial patch 3.

One embodiment comprises a myocardial patch and an injection system, wherein the injection system is in fluid communication with at least one of a bordering lumen and a reservoir lumen. The injection system is preferably charged with a carrier material to be filled into the reservoir lumen and/or one or more adhesive components to be filled into the bordering lumen. Alternatively or additionally, the injection system is connected to a reservoir of a carrier material to be filled into the reservoir lumen and/or one or more adhesive components to be filled into the bordering lumen. The injection system can comprise an infusion pump or an auto-injector for controlled injection of a carrier material into the reservoir lumen and/or one or more adhesive components into the bordering lumen. Therein, a controlled flow of carrier material or adhesive can be ensured. Further, a pre-determined pre-gelling timing of a carrier material or a pre-determined mixing ratio of components of adhesive can be maintained for optimized filling of the reservoir lumen and/or the bordering lumen. Using an injection pump or an auto-injector, materials too viscous to be reliably injected by hand can be injected.

Implantation of the myocardial patch 3 to a therapy site at the heart of the patient can be realized using a range of implantation techniques. The therapy site may be a heart surface, such as, the epicardial surface 2 or the inner surface of the pericardial sac 4, where the patch 3 is attached to when implanted on a heart 1.

According to one approach, a puncture needle having a center lumen is inserted into the mid-chest region via a subxiphoidal or intercostal approach. Subsequently, the puncture needle is introduced into the pericardial space through a puncture in the pericardial sac. Using a medical imaging method as described before, the entry of the puncture needle into the pericardial sac can be verified. Subsequently, the center lumen of the puncture needle is used to advance a guidewire into the pericardial space. The guidewire is used to advance a dilator and an introducer over the guidewire into the pericardial space and removed thereafter. At this point, the dilator can be removed with the guidewire. Eventually, the myocardial patch is introduced into the pericardial space and positioned as wished on the epicardial surface via the introducer and if needed, using a stylet.

According to another approach, a small surgical access window is made in the subxiphoidal or intercostal approach of between 2 to 10 cm are made, preferably between 4 and 6cm, in order to access the pericardium. Artificial collapsing of the adjacent lung might be necessary to enable direct visual access of the pericardium. Subsequently, an incision approximately the size of the surgical access can be made in the pericardium. Thereafter, whilst stabilizing the pericardium per standard surgical forceps or temporary stitch to the adjacent structures, the myocardial patch can be introduced per the appropriate delivery device, positioned correctly on the epicardial surface until securely adhere and the released from the delivery device.

Implantation of the myocardial patch to the epicardial surface of the heart is simplified, when a more-invasive procedure such as a sternotomy is applied. However, minimal-invasive procedures are usually preferred for smaller scars and a smaller risk of infections.

In one example, the carrier material containing the therapeutic agent is injected into the myocardial patch prior to implantation. Thereby, the gelation of the carrier material can be inspected before implanting the patch with the carrier material. Further, a risk of tearing the myocardial patch in situ during the injection is avoided.

In another example, the carrier material containing the therapeutic agent is injected into the myocardial patch after implantation. Thereby, the myocardial patch can be significantly smaller when it is initially implanted, facilitating the operation.

## Claims

1. A collapsible myocardial patch (3) for site-specific delivery of a therapeutic agent to an epicardial surface (2) of a heart (1), the myocardial patch (3) having a collapsed state (33a) and an expanded state (33b) and comprising:
a first layer (17) and a second layer (18) defining a delivery side (17a) and a cover side (18a) of the myocardial patch (3) in the expanded state (33b) of the patch (3);
at least one reservoir lumen (8), wherein the reservoir lumen (8) is defined by the first layer (17) and/or second layer (18) and configured to contain a therapeutic agent;
at least one bordering lumen (7) which is defined by the first layer (17) and/or second layer (18), wherein the bordering lumen (7) is different from the reservoir lumen (8) and configured to contain and release an adhesive material;
at least one port (9) which is in fluid communication with at least one of the bordering lumen (7) and configured to receive the adhesive material;
wherein the first layer (17) and the second layer (18) are configured to preferentially release the therapeutic agent towards the delivery side (17a) of the patch (3) in its expanded state (33b).

2. The collapsible myocardial patch (3) according to claim 1, wherein the at least one bordering lumen (7) is in a peripheral region on the patch (3).

3. The collapsible myocardial patch (3) according to any one of claims 1 or 2, wherein the at least one bordering lumen (7) is configured to release the adhesive material towards the delivery side (17a) of the patch (3) in its expanded state (33b).

4. The collapsible myocardial patch (3) according to claim 3, wherein the at least one bordering lumen (7) comprises one or more openings arranged on the delivery side (17a) of the patch (3) in its expanded state (33b).

5. The collapsible myocardial patch (3) according to any one of claims 1 to 4, wherein the first layer (17) is permeable to the therapeutic agent and wherein the second layer 18 is less permeable to the therapeutic agent than the first layer (17).

6. The collapsible myocardial patch (3) according to any one of claims 1 to 5, wherein
the first layer (17) is biodegradable under in-situ conditions and wherein the second layer (18) is more slowly biodegrading than the first layer (17) under said in-situ conditions.

7. The collapsible myocardial patch (3) according to any one of claims 1 to 6, wherein the first and a second layer (18) join adjacent to one or more bordering regions and define the reservoir lumen (8).

8. The collapsible myocardial patch (3) according to any one of claims 1 to 6, wherein the first and the second layer (18) are connected to one or more bordering regions and wherein the first and the second layer (18) and the one or more bordering regions define the reservoir lumen (8).

9. The collapsible myocardial patch (3) according to any one of claims 1 to 8, wherein the reservoir lumen (8) is in fluid communication with a second port which is configured to receive the therapeutic agent and a carrier material.

10. The collapsible myocardial patch (3) according to claim 4, wherein the one or more openings in the bordering lumen (7) are a plurality of holes, slits or pores which are configured to allow passage of the adhesive material.

11. The collapsible myocardial patch (3) according to any one of claims 1 to 10, wherein at least one of the bordering lumen (7) define one or more channels.

12. The collapsible myocardial patch (3) according to any one of claims 1 to 11, wherein at least part of its surface comprises a feature that facilitates tissue ingrowth and/or fibrosis.

13. The collapsible myocardial patch (3) according to claim 12, wherein said feature is one or more features selected from the group consisting of:
a) a layer of non-woven fibrous material that covers at least in part the surface area of the reservoir lumen (8),
b) a layer of non-woven fibrous material that covers at least in part the surface area of the patch adjacent to the reservoir lumen (8),
c) an irregular surface morphology that facilitates tissue ingrowth and/or fibrosis that covers at least in part the surface area of the reservoir lumen (8),
d) an irregular surface morphology that facilitates tissue ingrowth and/or fibrosis that covers at least in part the surface area of the patch adjacent to the reservoir lumen (8),
e) a layer of open- or closed-cell foam material that covers at least in part the surface area of the reservoir lumen (8),
f) a layer of open- or closed-cell foam material that covers at least in part the surface area of the patch adjacent to the reservoir lumen (8),
g) a second therapeutic agent that facilitates tissue ingrowth and/or fibrosis that covers at least in part the surface area of the reservoir lumen (8), and
h) a second therapeutic agent that facilitates tissue ingrowth and/or fibrosis that covers at least in part the surface area of the patch adjacent to the reservoir lumen (8).

14. The collapsible myocardial patch (3) according to any one of claims 1 to 13, wherein the patch is releasably attached to a support structure which provides one or more of the following features:
a) an inflatable member (31) which is configured to expand the myocardial patch (3) from its collapsed state into an expanded state,
b) a shape memory material which is configured to expand the myocardial patch (3) from its collapsed state into an expanded state,
c) a hollow member which is configured to receive a catheter wire or stylet which expands the myocardial patch (3) from its collapsed state into an expanded state, and
d) a flexible foam material which is configured to expand the myocardial patch (3) from its collapsed state into an expanded state.

15. The collapsible myocardial patch (3) according to any one of claims 1 to 14, wherein the patch is configured to allow its passage through a catheter or port having an internal diameter of less than about 15 mm, in particular less than about 11 mm, in its collapsed state.

## Patentansprüche

1. Faltbarer Myokard-Patch (3) für die ortsspezifische Zuführung eines Therapeutikums zu einer epikardialen Oberfläche (2) eines Herzes (1), wobei der Myokard-Patch (3) einen gefalteten Zustand (33a) und einen expandierten Zustand (33b) hat und Folgendes umfasst:
eine erste Schicht (17) und eine zweite Schicht (18), die eine Zuführungsseite (17a) und eine Abdeckungsseite (18a) des Myokard-Patch (3) in dem expandierten Zustand (33b) des Patch (3) definieren,
mindestens ein Reservoirlumen (8), wobei das Reservoirlumen (8) durch die erste Schicht (17) und/oder die zweite Schicht (18) definiert und dazu ausgestaltet ist, ein Therapeutikum zu enthalten,
mindestens ein angrenzendes Lumen (7), das durch die erste Schicht (17) und/oder die zweite Schicht (18) definiert ist, wobei das angrenzende Lumen (7) von dem Reservoirlumen (8) verschieden und dazu ausgestaltet ist, ein Haftmaterial zu enthalten und freizusetzen,
mindestens einen Port (9), der in Fluidverbindung mit mindestens einem des angrenzenden Lumens (7) ist und zur Aufnahme des Haftmaterials ausgestaltet ist,
wobei die erste Schicht (17) und die zweite Schicht (18) dazu ausgestaltet sind, das Therapeutikum vorzugsweise zu der Zuführseite (17a) des Patch (3) hin in seinem expandierten Zustand (33b) freizusetzen.

2. Faltbarer Myokard-Patch (3) nach Anspruch 1, wobei sich das mindestens eine angrenzende Lumen (7) in einem Umfangsbereich auf dem Patch (3) befindet.

3. Faltbarer Myokard-Patch (3) nach einem der Ansprüche 1 oder 2, wobei das mindestens eine angrenzende Lumen (7) zum Freisetzen des Haftmaterials zu der Zuführseite (17a) des Patch (3) hin in seinem expandierten Zustand (33b) ausgestaltet ist.

4. Faltbarer Myokard-Patch (3) nach Anspruch 3, wobei das mindestens eine angrenzende Lumen (7) eine oder mehrere Öffnungen umfasst, die an der Zuführseite (17a) des Patch (3) in seinem expandierten Zustand (33b) angeordnet sind.

5. Faltbarer Myokard-Patch (3) nach einem der Ansprüche 1 bis 4, wobei die erste Schicht (17) für das Therapeutikum durchlässig ist und wobei die zweite Schicht (18) für das Therapeutikum weniger durchlässig als die erste Schicht (17) ist.

6. Faltbarer Myokard-Patch (3) nach einem der Ansprüche 1 bis 5, wobei die erste Schicht (17) unter In-situ-Bedingungen biologisch abbaubar ist und wobei die zweite Schicht (18) unter den In-situ-Bedingungen biologisch langsamer als die erste Schicht (17) abgebaut wird.

7. Faltbarer Myokard-Patch (3) nach einem der Ansprüche 1 bis 6, wobei die erste und die zweite Schicht (18) in der Nähe von einem oder mehreren angrenzenden Bereichen zusammenkommen und das Reservoirlumen (8) definieren.

8. Faltbarer Myokard-Patch (3) nach einem der Ansprüche 1 bis 6, wobei die erste und die zweite Schicht (18) mit einem oder mehreren angrenzenden Bereichen verbunden sind und wobei die erste und die zweite Schicht (18) und der eine oder die mehreren angrenzenden Bereichen das Reservoirlumen (8) definieren.

9. Faltbarer Myokard-Patch (3) nach einem der Ansprüche 1 bis 8, wobei das Reservoirlumen (8) mit einem zweiten Port in Fluidverbindung ist, der zur Aufnahme des Therapeutikums und eines Trägermaterials ausgestaltet ist.

10. Faltbarer Myokard-Patch (3) nach Anspruch 4, wobei die eine oder die mehreren Öffnungen in dem angrenzenden Lumen (7) eine Vielzahl von Löchern, Schlitzen oder Poren sind, die dazu ausgestaltet sind, den Durchgang des Haftmaterials zu gestatten.

11. Faltbarer Myokard-Patch (3) nach einem der Ansprüche 1 bis 10, wobei mindestens eines des angrenzenden Lumens (7) einen oder mehrere Kanäle definiert.

12. Faltbarer Myokard-Patch (3) nach einem der Ansprüche 1 bis 11, wobei mindestens ein Teil seiner Oberfläche ein Merkmal umfasst, das Hineinwachsen von Gewebe und/oder Fibrose ermöglicht.

13. Faltbarer Myokard-Patch (3) nach Anspruch 12, wobei es sich bei dem Merkmal um ein oder mehrere Merkmale handelt, die aus Gruppe ausgewählt sind, die aus Folgendem besteht:
a) einer Schicht aus fasrigem Vliesmaterial, das die Oberfläche des Reservoirlumens (8) mindestens teilweise abdeckt,
b) einer Schicht aus fasrigem Vliesmaterial, das die Oberfläche des dem Reservoirlumen (8) benachbarten Patch mindestens teilweise abdeckt,
c) einer unregelmäßige Oberflächenmorphologie, die das Hineinwachsen von Gewebe und/oder Fibrose ermöglicht und die die Oberfläche des Reservoirlumens (8) mindestens teilweise abdeckt,
d) einer unregelmäßige Oberflächenmorphologie, die das Hineinwachsen von Gewebe und/oder Fibrose ermöglicht und die die Oberfläche des dem Reservoirlumen (8) benachbarten Patch mindestens teilweise abdeckt,
e) einer Schicht aus offen- oder geschlossenzelligem Schaummaterial, die die Oberfläche des Reservoirlumens (8) mindestens teilweise abdeckt,
f) einer Schicht aus offen- oder geschlossenzelligem Schaummaterial, die die Oberfläche des dem Reservoirlumen (8) benachbarten Patch mindestens teilweise abdeckt,
g) einem zweiten Therapeutikum, das das Hineinwachsen von Gewebe und/oder Fibrose ermöglicht und die Oberfläche des Reservoirlumens (8) mindestens teilweise abdeckt, und
h) einem zweiten Therapeutikum, das das Hineinwachsen von Gewebe und/oder Fibrose ermöglicht und die Oberfläche des dem Reservoirlumen (8) benachbarten Patch mindestens teilweise abdeckt.

14. Faltbarer Myokard-Patch (3) nach einem der Ansprüche 1 bis 13, wobei der Patch freigebbar an einer Stützstruktur angebracht ist, die eines oder mehrere der folgenden Merkmale bereitstellt:
a) ein inflatierbares Glied (31), das dazu ausgestaltet ist, den Myokard-Patch (3) aus seinem gefalteten Zustand in einen expandierten Zustand zu expandieren,
b) ein Formgedächtnismaterial, das dazu ausgestaltet ist, den Myokard-Patch (3) aus seinem gefalteten Zustand in einen expandierten Zustand zu expandieren,
c) ein hohles Glied, das zur Aufnahme eines Katheterdrahts oder Stiletts ausgestaltet ist und das den Myokard-Patch (3) aus seinem gefalteten Zustand in einen expandierten Zustand expandiert, und
d) ein flexibles Schaummaterial, das dazu ausgestaltet ist, den Myokard-Patch (3) aus seinem gefalteten Zustand in einen expandierten Zustand zu expandieren.

15. Faltbarer Myokard-Patch (3) nach einem der Ansprüche 1 bis 14, wobei der Patch so ausgestaltet ist, dass er in seinem gefalteten Zustand durch einen Katheter oder Port mit einem Innendurchmesser von weniger als ungefähr 15 mm, insbesondere weniger als ungefähr 11 mm, gehen kann.

## Revendications

1. Timbre myocardique pliable (3) pour l'administration spécifique au site d'un agent thérapeutique sur une surface épicardique (2) d'un cœur (1), le timbre myocardique (3) ayant un état replié (33a) et un état dilaté (33b) et comprenant :
une première couche (17) et une seconde couche (18) définissant un côté d'administration (17a) et un côté de recouvrement (18a) du timbre myocardique (3) dans l'état dilaté (33b) du timbre (3) ;
au moins une lumière de réservoir (8), la lumière de réservoir (8) étant définie par la première couche (17) et/ou la seconde couche (18) et configurée pour contenir un agent thérapeutique ;
au moins une lumière de bordure (7) définie par la première couche (17) et/ou la seconde couche (18), la lumière de bordure (7) étant différente de la lumière de réservoir (8) et configurée pour contenir et libérer un matériau adhésif ;
au moins un orifice (9) en communication fluidique avec au moins l'une des lumières de bordure (7) et configuré pour recevoir le matériau adhésif ;
la première couche (17) et la seconde couche (18) étant configurées pour libérer préférentiellement l'agent thérapeutique vers le côté d'administration (17a) du timbre (3) dans son état dilaté (33b).

2. Timbre myocardique pliable (3) selon la revendication 1, l'au moins une lumière de bordure (7) étant dans une région de bordure sur le timbre (3).

3. Timbre myocardique pliable (3) selon l'une quelconque des revendications 1 ou 2, l'au moins une lumière de bordure (7) étant configurée pour libérer le matériau adhésif vers le côté d'administration (17a) du timbre (3) dans son état dilaté (33b).

4. Timbre myocardique pliable (3) selon la revendication 3, l'au moins une lumière de bordure (7) comprenant une ou plusieurs ouvertures agencées sur le côté d'administration (17a) du timbre (3) dans son état dilaté (33b).

5. Timbre myocardique pliable (3) selon l'une quelconque des revendications 1 à 4, la première couche (17) étant perméable à l'agent thérapeutique et la seconde couche 18 étant moins perméable à l'agent thérapeutique que la première couche (17).

6. Timbre myocardique pliable (3) selon l'une quelconque des revendications 1 à 5, la première couche (17) étant biodégradable dans des conditions in situ, et la seconde couche (18) se dégradant plus lentement que la première couche (17) dans lesdites conditions in situ.

7. Timbre myocardique pliable (3) selon l'une quelconque des revendications 1 à 6, la première et la seconde couche (18) réunissant de manière adjacente une ou plusieurs régions de bordure, et définissant la lumière de réservoir (8).

8. Timbre myocardique pliable (3) selon l'une quelconque des revendications 1 à 6, la première et la seconde couche (18) étant reliées à une ou plusieurs régions de bordure, et la première et la seconde couche (18) et la ou les régions de bordure définissant la lumière de réservoir (8).

9. Timbre myocardique pliable (3) selon l'une quelconque des revendications 1 à 8, la lumière de réservoir (8) étant en communication fluidique avec un second orifice configuré pour recevoir l'agent thérapeutique et un matériau porteur.

10. Timbre myocardique pliable (3) selon la revendication 4, la ou les ouvertures dans la lumière de bordure (7) étant une pluralité de trous, de fentes ou de pores qui sont configurés pour permettre le passage du matériau adhésif.

11. Timbre myocardique pliable (3) selon l'une quelconque des revendications 1 à 10, au moins une des lumières de bordure (7) définissant un ou plusieurs canaux.

12. Timbre myocardique pliable (3) selon l'une quelconque des revendications 1 à 11, au moins une partie de sa surface comprenant une caractéristique qui facilite la croissance des tissus et/ou la fibrose.

13. Timbre myocardique pliable (3) selon la revendication 12, ladite caractéristique étant une ou plusieurs caractéristiques choisies dans le groupe constitué par :
a) une couche de matériau fibreux non tissé qui recouvre au moins en partie la surface de la lumière de réservoir (8),
b) une couche de matériau fibreux non tissé qui recouvre au moins en partie la surface du timbre adjacente à la lumière de réservoir (8),
c) une morphologie de surface irrégulière qui facilite la croissance des tissus et/ou la fibrose qui recouvre au moins en partie la surface de la lumière de réservoir (8),
d) une morphologie de surface irrégulière qui facilite la croissance des tissus et/ou la fibrose qui recouvre au moins en partie la surface du timbre adjacent à la lumière de réservoir (8),
e) une couche de mousse à cellules ouvertes ou fermées qui recouvre au moins en partie la surface de la lumière de réservoir (8),
f) une couche de mousse à cellules ouvertes ou fermées qui recouvre au moins en partie la surface du timbre adjacent à la lumière de réservoir (8),
g) un second agent thérapeutique qui facilite la croissance des tissus et/ou la fibrose et qui recouvre au moins en partie la surface de la lumière de réservoir (8), et
h) un second agent thérapeutique qui facilite la croissance des tissus et/ou la fibrose et qui recouvre au moins en partie la surface du timbre adjacent à la lumière de réservoir (8).

14. Timbre myocardique pliable (3) selon l'une quelconque des revendications 1 à 13, le timbre étant fixé de manière amovible à une structure de support qui fournit une ou plusieurs des caractéristiques suivantes :
a) un élément gonflable (31) qui est configuré pour dilater le timbre myocardique (3) de son état replié à un état dilaté,
b) un matériau à mémoire de forme qui est configuré pour dilater le timbre myocardique (3) de son état replié à un état dilaté,
c) un élément creux qui est configuré pour recevoir un fil ou un stylet de cathéter qui dilate le timbre myocardique (3) de l'état replié à l'état dilaté, et
d) un matériau en mousse souple qui est configuré pour dilater le timbre myocardique (3) de l'état replié à l'état dilaté.

15. Timbre myocardique pliable (3) selon l'une quelconque des revendications 1 à 14, le timbre étant configuré pour permettre son passage à travers un cathéter ou un orifice ayant un diamètre interne inférieur à environ 15 mm, en particulier inférieur à environ 11 mm, à l'état replié.
